# EUROPEAN PATENT APPLICATION

(11) **EP 3 620 211 A1**
(43) Date of publication of application: **11.03.2020**
(21) Application number: 19190296.4
(22) Date of filing: 01.06.2012
(51) Int. Cl.: A61P 17/02, A61K 31/352, A61P 43/00

(54) **TETRAHYDROCANNABINOL-11-OIC ACIDS FOR USE IN METHODS OF TREATING FIBROTIC DISEASES**

(30) Priority: 04.06.2011 US 201161493435 P; 31.05.2012 US 201213485044
(62) Divisional of application: 12796691.9
(71) Applicant: Corbus Pharmaceuticals, Inc., Norwood, MA 02062 (US)
(72) Inventor: ZURIER, Robert B., Wyckoff, NJ 07481 (US); SELVI, Enrico, 53011 Castellina in Chianti-Siena (IT)
(74) Representative: Cabinet Becker et Associés

(57) **Abstract**

This invention is in the field of medicinal chemistry and relates to novel compounds, and pharmaceutical compositions and methods of use thereof for the treatment and/or prevention of fibrotic diseases including scleroderma, systemic sclerosis, scleroderma-like disorders, sine scleroderma, liver cirrhosis, interstitial pulmonary fibrosis, Dupuytren's contracture, keloids, chronic kidney disease, chronic graft rejection, and other scarring/wound healing abnormalities, post operative adhesions, and reactive fibrosis. The invention also relates to methods of using the compounds and pharmaceutical compositions of this invention to treat fibrotic conditions.

## Description

### CROSS-REFERENCE TO RELATED APPLICATIONS

The present application claims the benefit of U.S. Provisional Patent Application No. 61/493,435 filed June 4, 2011.

### FIELD OF THE INVENTION

This invention is in the field of medicinal chemistry and relates to novel compounds, and pharmaceutical compositions and methods of use thereof for the treatment and/or prevention of fibrotic diseases including but not limited to scleroderma, systemic sclerosis, scleroderma-like disorders, sine scleroderma, liver cirrhosis, interstitial pulmonary fibrosis, Dupuytren's contracture, keloids, chronic kidney disease, chronic graft rejection, and other scarring/wound healing abnormalities, post operative adhesions, and reactive fibrosis. The invention also relates to methods of using the compounds and pharmaceutical compositions of this invention to treat fibrotic conditions.

### BACKGROUND OF THE INVENTION

Fibrotic diseases, including pulmonary fibrosis, systemic sclerosis, liver cirrhosis, cardiovascular disease, progressive kidney disease, and macular degeneration, are a leading cause of morbidity and mortality and can affect all tissues and organ systems. Fibrotic tissue remodeling can also influence cancer metastasis and accelerate chronic graft rejection in transplant recipients. Nevertheless, despite its enormous impact on human health, there are currently no approved treatments that directly target the mechanism(s) of fibrosis.

### SUMMARY OF THE INVENTION

This invention is in the field of medicinal chemistry and relates to novel compounds, and pharmaceutical compositions and methods of use thereof for the treatment and/or prevention of fibrotic diseases including but not limited to scleroderma, systemic sclerosis, scleroderma-like disorders, sine scleroderma, liver cirrhosis, interstitial pulmonary fibrosis, Dupuytren's contracture, keloids, chronic kidney disease, chronic graft rejection, and other scarring/wound healing abnormalities, post operative adhesions, and reactive fibrosis. The invention also relates to methods of using the compounds and pharmaceutical compositions of this invention to treat fibrotic conditions.

In one embodiment, the present invention relates to the use of non-psychotropic 11-oic acid derivatives of tetrahydrocannabinol of formula (I) for the treatment and/or prevention of fibrotic diseases including scleroderma, systemic sclerosis, scleroderma-like disorders, sine scleroderma, liver cirrhosis, interstitial pulmonary fibrosis, Dupuytren's contracture, keloids, chronic kidney disease, and other scarring/wound healing abnormalities, post operative adhesions, and reactive fibrosis. In particular the present invention relates to the use of (3R,4R)-Δ8-tetrahydrocannabinol-11-oic acids, and pharmaceutical compositions comprising therapeutically effective amounts of the acids in all forms for the treatment of scleroderma and other fibrotic diseases.

In one embodiment, the invention relates to a method, comprising: a) providing: i) a subject exhibiting at least one symptom of fibrotic disease; ii) a composition comprising ajulemic acid; b) administering said composition to said subject; and c) reducing said at least one symptom of fibrotic disease. In one embodiment, said fibrotic disease is dermal fibrosis and said symptom is dermal thickening. In one embodiment, said fibrotic disease is lung fibrosis and said symptom is leukocyte infiltration. In one embodiment, said fibrotic disease is selected from the group consisting of scleroderma, systemic sclerosis, scleroderma-like disorders, sine scleroderma, liver cirrhosis, interstitial pulmonary fibrosis, Dupuytren's contracture, keloids, chronic kidney disease, chronic graft rejection, and other scarring/wound healing abnormalities, post operative adhesions, and reactive fibrosis. In one embodiment, said composition is administered orally. In one embodiment, said composition is administered intravenously. In one embodiment, said composition is administered via an implant or patch. In one embodiment, said implant or patch provides slow release of said composition. In one embodiment, said composition is administered by inhalation. In one embodiment, said composition is administered in a tablet.

In one embodiment, the present invention relates to a method, comprising: a) providing: i) a subject exhibiting at least one symptom of fibrotic disease; ii) a composition comprising a therapeutically effective amount of a (3R,4R)-Δ8-tetrahydrocannabinol-11-oic acid of the formula (I); b) administering said composition to said subject; and c) reducing in said subject at least one symptom of fibrotic disease. In one embodiment, said formula (I) is: 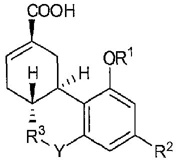 wherein R¹ is hydrogen, COCH₃ or COCH₂CH₃; R² is a branched C5-C12 alkyl group which may optionally have a terminal aromatic ring, or optionally a branched OCHCH₃(CH₂)ₘ alkyl group which may have a terminal aromatic ring, wherein m is 0 to 7; and R³ is hydrogen, a C1-8 alkyl or a C1-8 alkanol group; and Y is nil or a bridging group of NH or oxygen; provided that where Y is oxygen and R₂ is a branched C5-C12 alkyl, R³ is not CHCH₃. In one embodiment, said composition comprises a pharmaceutically acceptable salt, ester, or solvate of (3R,4R)-Δ8-tetrahydrocannabinol-11-oic acid. In one embodiment, said fibrotic disease comprises scleroderma, systemic sclerosis, scleroderma-like disorders, sine scleroderma, liver cirrhosis, interstitial pulmonary fibrosis, Dupuytren's contracture, keloids, chronic kidney disease, chronic graft rejection, and other scarring/wound healing abnormalities, post operative adhesions, and reactive fibrosis. In one embodiment, R¹ is hydrogen and R² is 1',1'- dimethylheptyl. In one embodiment, said composition has the structure: 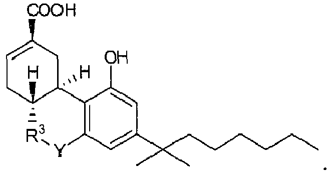 In one embodiment, R² is a branched OCHCH₃(CH₂)ₘ alkyl group terminated with a phenyl ring, wherein m is 0 to 7, and R³ is CHCH₃. In one embodiment, said composition has the structure: 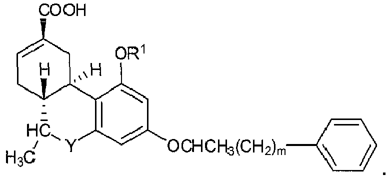 In one embodiment, said composition is administered orally. In one embodiment, said composition is administered intravenously. In one embodiment, said composition is administered via an implant or patch. In one embodiment, said implant or patch provides slow release of the composition. In one embodiment, said composition is administered by inhalation. In one embodiment, said composition is administered in a tablet.

In one embodiment, the present invention relates to a method, comprising: a) providing: i) a subject exhibiting at least one symptom of fibrotic disease; ii) a composition comprising a therapeutically effective amount of a (3R,4R)-Δ8-tetrahydrocannabinol-11-oic acid is (6aR,10aR)-4-(1,1-dimethylheptyl)-Δ8-tetrahydro-cannabinol-9-carboxylic acid of the formula (II); b) administering said composition to said subject; and c) reducing said at least one symptom of fibrotic disease. In one embodiment, said formula (II) is: 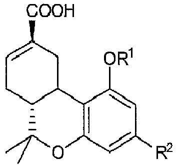 wherein R¹ is hydrogen, COCH₃ or COCH₂CH₃; and R² is a branched C5-C12 alkyl group which may optionally have a terminal aromatic ring, or optionally a branched OCHCH₃(CH₂)ₘ alkyl group which may have a terminal aromatic ring, wherein m is 0 to 7. In one embodiment, said composition comprises a pharmaceutically acceptable salt, ester, or solvate of (3R,4R)-Δ8-tetrahydrocannabinol-11-oic acid is (6aR,10aR)-4-(1,1-dimethylheptyl)-Δ8-tetrahydro-cannabinol-9-carboxylic acid. In one embodiment, said fibrotic disease comprises scleroderma, systemic sclerosis, scleroderma-like disorders, sine scleroderma, liver cirrhosis, interstitial pulmonary fibrosis, Dupuytren's contracture, keloids, chronic kidney disease, chronic graft rejection, and other scarring/wound healing abnormalities, post operative adhesions, and reactive fibrosis. In one embodiment, R¹ is hydrogen and R² is 1',1'- dimethylheptyl. In one embodiment, R² is a branched OCHCH₃(CH₂)ₘ alkyl group terminated with a phenyl ring, wherein m is 0 to 7, and R³ is CHCH₃. In one embodiment, said composition is administered orally. In one embodiment, said composition is administered intravenously. In one embodiment, said composition is administered via an implant or patch. In one embodiment, said implant or patch provides slow release of said composition. In one embodiment, said composition is administered by inhalation. In one embodiment, said composition is administered in a tablet.

The present invention relates to the use of 11-oic acid derivatives of tetrahydrocannabinol of formula (I) 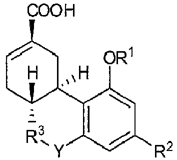 wherein R¹ is hydrogen, COCH₃ or COCH₂CH₃; R² is a branched C5-C12 alkyl group which may optionally have a terminal aromatic ring, or optionally a branched OCHCH₃(CH₂)ₘ alkyl group which may have a terminal aromatic ring, wherein m is 0 to 7; R³ is hydrogen, a C1-8 alkyl or a C1-8 alkanol group; and Y is nil or a bridging group of NH or oxygen, provided that where Y is oxygen and R² is a branched C5-C12 alkyl, R³ is not CHCH₃, and pharmaceutically acceptable salts, esters, or solvate thereof for the treatment and/or prevention of fibrotic diseases including scleroderma, systemic sclerosis, scleroderma-like disorders, sine scleroderma, liver cirrhosis, interstitial pulmonary fibrosis, Dupuytren's contracture, keloids, chronic kidney disease, chronic graft rejection, and other scarring/wound healing abnormalities, post operative adhesions, and reactive fibrosis. In particular the present invention relates to the use of (3R,4R)-Δ 8-tetrahydrocannabinol-11-oic acids, and pharmaceutical compositions comprising therapeutically effective amounts of the acids in all forms for the treatment of scleroderma and other fibrotic diseases.

In one embodiment, the invention relates to a method of preventing and/or treating fibrotic diseases including scleroderma, systemic sclerosis, scleroderma-like disorders, sine scleroderma, liver cirrhosis, interstitial pulmonary fibrosis, Dupuytren's contracture, keloids, chronic kidney disease, chronic graft rejection, and other scarring/wound healing abnormalities, post operative adhesions, and reactive fibrosis.comprising administering to a subject in need thereof, a composition that comprises a therapeutically effective amount of a (3R,4R)-Δ8-tetrahydrocannabinol-11-oic acid of the formula (I) or its tautomers, its geometrical isomers, its optically active forms as enantiomers, diastereomers and its racemate forms, pharmaceutically acceptable salts thereof, polymorphs and combinations thereof, to said subject. 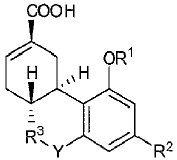 wherein R¹ is hydrogen, COCH₃ or COCH₂CH₃; R₂ is a branched C5-C12 alkyl group which may optionally have a terminal aromatic ring, or optionally a branched OCHCH₃(CH₂)ₘ alkyl group which may have a terminal aromatic ring, wherein m is 0 to 7; and R₃ is hydrogen, a C1-8 alkyl or a C1-8 alkanol group; and Y is nil or a bridging group of NH or oxygen; provided that where Y is oxygen and R² is a branched C5-C12 alkyl, R³ is not CHCH₃, or a pharmaceutically acceptable salt, ester, or solvate thereof, the method comprising: In one embodiment, R₁ is hydrogen and R₂ is 1', 1'- dimethylheptyl. In one embodiment, R₂ is a branched OCHCH₃(CH₂)ₘ alkyl group terminated with a phenyl ring, wherein m is 0 to 7, and R₃ is CHCH₃. In one embodiment, the compound is administered orally. In one embodiment, the compound is administered intravenously. In one embodiment, the compound is administered via an implant or patch. In one embodiment, the implant or patch provides slow release of the compound. In one embodiment, the compound is administered by inhalation. In one embodiment, the compound is administered in a tablet.

In one embodiment, the (3R,4R)-Δ8-tetrahydrocannabinol-11-oic acid is (6aR,10aR)-4-(1,1-dimethylheptyl)-Δ8-tetrahydro-cannabinol-9-carboxylic acid or its tautomers, its geometrical isomers, its optically active forms as enantiomers, diastereomers and its racemate forms, pharmaceutically acceptable salts thereof, polymorphs, and combinations thereof. In one embodiment, the compound is administered orally. In one embodiment, the compound is administered intravenously. In one embodiment, the compound is administered via an implant or patch. In one embodiment, the implant or patch provides slow release of the compound. In one embodiment, the compound is administered by inhalation. In one embodiment, the compound is administered in a tablet.

### DEFINITIONS

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims.

Compounds or compositions described are also to include, in some embodiments, its tautomers, its geometrical isomers, its optically active forms as enantiomers, diastereomers and its racemate forms, pharmaceutically acceptable salts thereof, polymorphs and combinations thereof.

The term "epimers", as used herein, refer to diastereomers that differ in configuration of only one stereogenic center. Diastereomers are a class of stereoisomers that are non-superposable, non-mirror images of one another, unlike enantiomers that are non-superposable mirror images of one another.

The term "common carriers", as used herein, refers to those which are employed in standard pharmaceutical preparations and includes excipients, binders and disintegrators the choice of which depends on the specific dosage form used. Typical examples of the excipient are starch, lactose, sucrose, glucose, mannitol and cellulose; illustrative binders are polyvinylpyrrolidone, starch, sucrose, hydroxypropyl cellulose and gum arabic; illustrative disintegrators include starch, agar, gelatin powder, cellulose, and CMC. Any other common excipients, binders and disintegrators may also be employed.

Formulations of the pharmaceutical composition of the present invention which are suitable for peroral administration may be provided in the form of tablets, capsules, powders, granules, or suspensions in non-aqueous solutions such as syrups, emulsions or drafts, each containing one or more of the active compounds in predetermined amounts.

The granule may be provided by first preparing an intimate mixture of one or more of the active ingredients with one or more of the auxiliary components shown above, then granulating the mixture, and classifying the granules by screening through a sieve.

The tablet may be prepared by compressing or otherwise forming one or more of the active ingredients, optionally with one or more auxiliary components.

The capsule may be prepared by first making a powder or granules as an intimate mixture of one or more of the active ingredients with one or more auxiliary components, then charging the mixture into an appropriate capsule on a packing machine, etc.

The pharmaceutical composition of the present invention may be formulated as a suppository (for rectal administration) with the aid of a common carrier such a cocoa butter. The pharmaceutical composition of the present invention may also be formulated in a dosage form suitable for non-parenteral administration by packaging one or more active ingredients as dry solids in a sterile nitrogen-purged container. The resulting dry formulation may be administered to patients non-parenterally after being dispersed or dissolved in a given amount of aseptic water.

If desired, the formulations may further contain one or more auxiliary components selected from among excipients, buffers, flavoring agents, binders, surfactants, thickening agents, and lubricants.

The dose will of course vary with the route of administration, the severity of the disease to be treated, and the patient to be treated, but the exact dose ultimately chosen should be left to the good discretion of the doctor responsible for the treatment. If a desired dose is determined, the active ingredient may be administered once a day or, alternatively, it may be administered in up to as many portions as deemed appropriate at suitable intervals. The active ingredient may be straightforwardly administered without being mixed with any other components. However, for several reasons, typically for the purpose of providing ease in controlling the dose level, the active compound is preferably administered in a pharmaceutical dosage form.

The term "salts", as used herein, refers to any salt that complexes with identified compounds contained herein while retaining a desired function, e.g., biological activity. Examples of such salts include, but are not limited to, acid addition salts formed with inorganic acids (e.g. hydrochloric acid, hydrobromic acid, sulfuric acid, phosphoric acid, nitric acid, and the like), and salts formed with organic acids such as, but not limited to, acetic acid, oxalic acid, tartaric acid, succinic acid, malic acid, fumaric acid, maleic acid, ascorbic acid, benzoic acid, tannic acid, pamoic acid, alginic acid, polyglutamic, acid, naphthalene sulfonic acid, naphthalene disulfonic acid, and polygalacturonic acid. Pharmaceutically acceptable salts also include base addition salts which may be formed when acidic protons present are capable of reacting with inorganic or organic bases. Suitable pharmaceutically-acceptable base addition salts include metallic salts, such as salts made from aluminum, calcium, lithium, magnesium, potassium, sodium and zinc, or salts made from organic bases including primary, secondary and tertiary amines, substituted amines including cyclic amines, such as caffeine, arginine, diethylamine, N-ethyl piperidine, histidine, glucamine, isopropylamine, lysine, morpholine, N-ethyl morpholine, piperazine, piperidine, triethylamine, trimethylamine. All of these salts may be prepared by conventional means from the corresponding compound of the invention by reacting, for example, the appropriate acid or base with the compound of the invention. Unless otherwise specifically stated, the present invention contemplates, in some embodiments, pharmaceutically acceptable salts of the specified compounds.

The term "alkyl" when used without the "substituted" modifier refers to a non-aromatic monovalent group with a saturated carbon atom as the point of attachment, a linear or branched, cyclo, cyclic or acyclic structure, no carbon-carbon double or triple bonds, and no atoms other than carbon and hydrogen. The groups, -CH₃ (Me), -CH₂CH₃ (Et), -CH₂CH₂CH₃ (*n*-Pr), -CH(CH₃)₂ (*iso*-Pr or *i*-Pr), -CH(CH₂)₂ (cyclopropyl), -CH₂CH₂CH₂CH₃ (*n*-Bu), -CH(CH₃)CH₂CH₃ (sec-butyl or *sec*-Bu), -CH₂CH(CH₃)₂ (*iso*-butyl or *i*-Bu), -C(CH₃)₃ (*tert*-butyl or *t*-Bu), -CH₂C(CH₃)₃ (neo-pentyl), cyclobutyl, cyclopentyl, cyclohexyl, cyclohexylmethyl are non-limiting examples of alkyl groups. The term "substituted alkyl" refers to a non-aromatic monovalent group with a saturated carbon atom as the point of attachment, a linear or branched, cyclo, cyclic or acyclic structure, no carbon-carbon double or triple bonds, and at least one atom independently selected from the group consisting of N, O, F, Cl, Br, I, Si, P, and S. The following groups are non-limiting examples of substituted alkyl groups: -CH₂OH, -CH₂Cl, -CH₂Br, -CH₂SH, -CF₃, -CH₂CN, -CH₂C(O)H, -CH₂C(O)OH, -CH₂C(O)OCH₃, -CH₂C(O)NH₂, -CH₂C(O)NHCH₃, -CH₂C(O)CH₃, -CH₂OCH₃, -CH₂OCH₂CF₃, -CH₂OC(O)CH₃, -CH₂NH₂, -CH₂NHCH₃, -CH₂N(CH₃)₂, -CH₂CH₂Cl, -CH₂CH₂OH, -CH₂CF₃, -CH₂CH₂OC(O)CH₃, -CH₂CH₂NHCO₂C(CH₃)₃, and -CH₂Si(CH₃)₃.

The term "alkanol" refers to any of a class of organic compounds containing the hydroxyl (-OH) functional group except those in which the OH group is attached to an aromatic ring (phenols).

The term "aryl" when used without the "substituted" modifier refers to a monovalent group with an aromatic carbon atom as the point of attachment, said carbon atom forming part of a six-membered aromatic ring structure wherein the ring atoms are all carbon, and wherein the monovalent group consists of no atoms other than carbon and hydrogen. Non-limiting examples of aryl groups include phenyl (Ph), methylphenyl, C₆H₃(CH₃)₂ (dimethylphenyl), -C₆H₄CH₂CH₃ (ethylphenyl), -C₆H₄CH₂CH₂CH₃ (propylphenyl), -C₆H₄CH(CH₃)₂, -C₆H₄CH(CH₂)₂, -C₆H₃(CH₃)CH₂CH₃ (methylethylphenyl), -C₆H₄CH=CH₂ (vinylphenyl), -C₆H₄CH=CHCH₃, -C₆H₄C≡CH, -C₆H₄C≡CCH₃, naphthyl, and the monovalent group derived from biphenyl. The term "substituted aryl" refers to a monovalent group with an aromatic carbon atom as the point of attachment, said carbon atom forming part of a six-membered aromatic ring structure wherein the ring atoms are all carbon, and wherein the monovalent group further has at least one atom independently selected from the group consisting of N, O, F, Cl, Br, I, Si, P, and S. Non-limiting examples of substituted aryl groups include the groups: -C₆H₄F, -C₆H₄Cl, -C₆H₄Br, -C₆H₄I, -C₆H₄OH, -C₆H₄OCH₃, -C₆H₄OCH₂CH₃, -C₆H₄OC(O)CH₃, -C₆H₄NH₂, -C₆H₄NHCH₃, -C₆H₄N(CH₃)₂, -C₆H₄CH₂OH, -C₆H₄CH₂OC(O)CH₃, -C₆H₄CH₂NH₂, -C₆H₄CF₃, -C₆H₄CN, -C₆H₄CHO, -C₆H₄CHO, -C₆H₄C(O)CH₃, -C₆H₄C(O)C₆H₅, -C₆H₄CO₂H, -C₆H₄CO₂CH₃, -C₆H₄CONH₂, -C₆H₄CONHCH₃, and -C₆H₄CON(CH₃)₂.

In addition, atoms making up the compounds of the present invention are intended to include all isotopic forms of such atoms. Isotopes, as used herein, include those atoms having the same atomic number but different mass numbers. By way of general example and without limitation, isotopes of hydrogen include tritium and deuterium, and isotopes of carbon include ¹³C and ¹⁴C. Similarly, it is contemplated that one or more carbon atom(s) of a compound of the present invention may be replaced by a silicon atom(s). Furthermore, it is contemplated that one or more oxygen atom(s) of a compound of the present invention may be replaced by a sulfur or selenium atom(s).

In structures wherein stereochemistry is not explicitly indicated, it is assumed that either stereochemistry is considered and both isomers claimed.

Any undefined valency on an atom of a structure shown in this application implicitly represents a hydrogen atom bonded to the atom.

The term "effective," as that term is used in the specification and/or claims, means adequate to accomplish a desired, or hoped for result.

An "isomer" of a first compound is a separate compound in which each molecule contains the same constituent atoms as the first compound, but where the configuration of those atoms in three dimensions differs.

As used herein, the term "patient" or "subject" refers to a living mammalian organism, such as a human, monkey, cow, sheep, goat, dog, cat, mouse, rat, guinea pig, or transgenic species thereof. In certain embodiments, the patient or subject is a primate. Non-limiting examples of human subjects are adults, juveniles, infants and fetuses.

The term "Pharmaceutically acceptable" means that which is useful in preparing a pharmaceutical composition that is generally safe, non-toxic and neither biologically nor otherwise undesirable and includes that which is acceptable for veterinary use as well as human pharmaceutical use.

"Pharmaceutically acceptable salts" means salts of compounds of the present invention which are pharmaceutically acceptable, as defined above, and which possess the desired pharmacological activity. Such salts include acid addition salts formed with inorganic acids such as hydrochloric acid, hydrobromic acid, sulfuric acid, nitric acid, phosphoric acid, and the like; or with organic acids such as 1,2-ethanedisulfonic acid, 2-hydroxyethanesulfonic acid, 2-naphthalenesulfonic acid, 3-phenylpropionic acid, 4,4'-methylenebis(3-hydroxy-2-ene-1 -carboxylic acid), 4-methylbicyclo[2.2.2]oct-2-ene-1-carboxylic acid, acetic acid, aliphatic mono- and dicarboxylicacids, aliphatic sulfuric acids, aromatic sulfuric acids, benzenesulfonic acid, benzoic acid, camphorsulfonic acid, carbonic acid, cinnamic acid, citric acid, cyclopentanepropionic acid, ethanesulfonic acid, fumaric acid, glucoheptonic acid, gluconic acid, glutamic acid, glycolic acid, heptanoic acid, hexanoic acid, hydroxynaphthoic acid, lactic acid, laurylsulfuric acid, maleic acid, malic acid, malonic acid, mandelic acid, methanesulfonic acid, muconic acid, *o*-(4-hydroxybenzoyl)benzoic acid, oxalic acid, *p*-chlorobenzenesulfonic acid, phenyl-substituted alkanoic acids, propionic acid, p-toluenesulfonic acid, pyruvic acid, salicylic acid, stearic acid, succinic acid, tartaric acid, tertiarybutylacetic acid, trimethylacetic acid, and the like. Pharmaceutically acceptable salts also include base addition salts which may be formed when acidic protons present are capable of reacting with inorganic or organic bases. Acceptable inorganic bases include sodium hydroxide, sodium carbonate, potassium hydroxide, aluminum hydroxide and calcium hydroxide. Acceptable organic bases include ethanolamine, diethanolamine, triethanolamine, tromethamine, *N*-metliylglucamine and the like. It should be recognized that the particular anion or cation forming a part of any salt of this invention is not critical, so long as the salt, as a whole, is pharmacologically acceptable. Additional examples of pharmaceutically acceptable salts and their methods of preparation and use are presented in Handbook of Pharmaceutical Salts: Properties, and Use (P. H. Stahl & C. G. Wermuth eds., Verlag Helvetica Chimica Acta, 2002) [1].

As used herein, "predominantly one enantiomer" means that a compound contains at least about 85% of one enantiomer, or more preferably at least about 90% of one enantiomer, or even more preferably at least about 95% of one enantiomer, or most preferably at least about 99% of one enantiomer. Similarly, the phrase "substantially free from other optical isomers" means that the composition contains at most about 15% of another enantiomer or diastereomer, more preferably at most about 10% of another enantiomer or diastereomer, even more preferably at most about 5% of another enantiomer or diastereomer, and most preferably at most about 1% of another enantiomer or diastereomer.

"Prevention" or "preventing" includes: (1) inhibiting the onset of a disease in a subject or patient which may be at risk and/or predisposed to the disease but does not yet experience or display any or all of the pathology or symptomatology of the disease, and/or (2) slowing the onset of the pathology or symptomatology of a disease in a subject or patient which may be at risk and/or predisposed to the disease but does not yet experience or display any or all of the pathology or symptomatology of the disease.

A "stereoisomer" or "optical isomer" is an isomer of a given compound in which the same atoms are bonded to the same other atoms, but where the configuration of those atoms in three dimensions differs. "Enantiomers" are stereoisomers of a given compound that are mirror images of each other, like left and right hands. "Diastereomers" are stereoisomers of a given compound that are not enantiomers.

Enantiomers are compounds that individually have properties said to have "optical activity" and consist of molecules with at least one chiral center, almost always a carbon atom. If a particular compound is dextrorotary, its enantiomer will be levorotary, and vice-versa. In fact, the enantiomers will rotate polarized light the same number of degrees, but in opposite directions. "Dextrorotation" and "levorotation" (also spelled laevorotation) refer, respectively, to the properties of rotating plane polarized light clockwise (for dextrorotation) or counterclockwise (for levorotation). A compound with dextrorotation is called "dextrorotary," while a compound with levorotation is called "levorotary".

A standard measure of the degree to which a compound is dextrorotary or levorotary is the quantity called the "specific rotation" "[α]". Dextrorotary compounds have a positive specific rotation, while levorotary compounds have negative. Two enantiomers have equal and opposite specific rotations. A dextrorotary compound is prefixed "(+)-" or "d-". Likewise, a levorotary compound is often prefixed "(-)-" or "1-". These "d-" and "1-" prefixes should not be confused with the "D-" and "L-" prefixes based on the actual configuration of each enantiomer, with the version synthesized from naturally occurring (+)-compound being considered the D-form. A mixture of enantiomers of the compounds is prefixed "(±)-". An equal mixture of enantiomers of the compounds is considered "optically inactive".

The invention contemplates that for any stereocenter or axis of chirality for which stereochemistry has not been defined, that stereocenter or axis of chirality can be present in its R form, S form, or as a mixture of the R and S forms, including racemic and non-racemic mixtures.

The present invention contemplates the above-described compositions in "therapeutically effective amounts" or "pharmaceutically effective amounts", which means that amount which, when administered to a subject or patient for treating a disease, is sufficient to effect such treatment for the disease or to ameliorate or reduce one or more symptoms of a disease or condition (e.g. reduce dermal thickening).

As used herein, the terms "treat" and "treating" are not limited to the case where the subject (e.g. patient) is cured and the disease is eradicated. Rather, the present invention also contemplates treatment that merely reduces symptoms, improves (to some degree) and/or delays disease progression. It is not intended that the present invention be limited to instances wherein a disease or affliction is cured. It is sufficient that symptoms are reduced.

In a specific embodiment, the term "pharmaceutically acceptable" means approved by a regulatory agency of the federal or a state government or listed in the U.S. Pharmacopeia or other generally recognized pharmacopeia for use in animals, and more particularly in humans. The term "carrier" refers to a diluent, adjuvant, excipient or vehicle with which the active compound is administered. Such pharmaceutical vehicles can be liquids, such as water and oils, including those of petroleum, animal, vegetable or synthetic origin, such as peanut oil, soybean oil, mineral oil, sesame oil and the like. The pharmaceutical vehicles can be saline, gum acacia, gelatin, starch paste, talc, keratin, colloidal silica, urea, and the like. In addition, auxiliary, stabilizing, thickening, lubricating and coloring agents can be used. When administered to a subject, the pharmaceutically acceptable vehicles are preferably sterile. Water can be the vehicle when the active compound is administered intravenously. Saline solutions and aqueous dextrose and glycerol solutions can also be employed as liquid vehicles, particularly for injectable solutions. Suitable pharmaceutical vehicles also include excipients such as starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol and the like. The present compositions, if desired, can also contain minor amounts of wetting or emulsifying agents, or pH buffering agents.

Pharmaceutically acceptable sugars include but are not limited to sucrose, dextrose, maltose, galactose, rhamnose, and lactose. Pharmaceutically acceptable sugar alcohols include but are not limited to mannitol, xylitol, and sorbitol.

### DESCRIPTION OF THE FIGURES

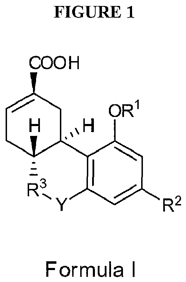

Figure 1 shows the formula for (3R,4R)-Δ8-tetrahydrocannabinol-11-oic acid derivatives.

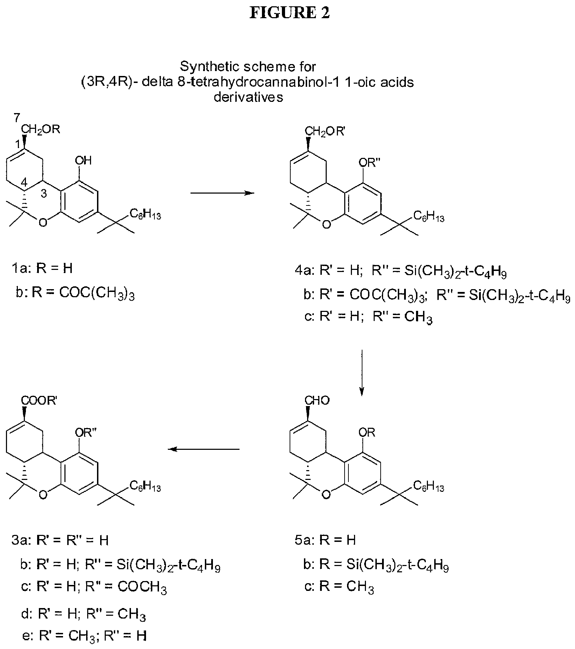

Figure 2 shows a synthetic scheme for (3R,4R)-Δ8-tetrahydrocannabinol-11-oic acids derivatives.

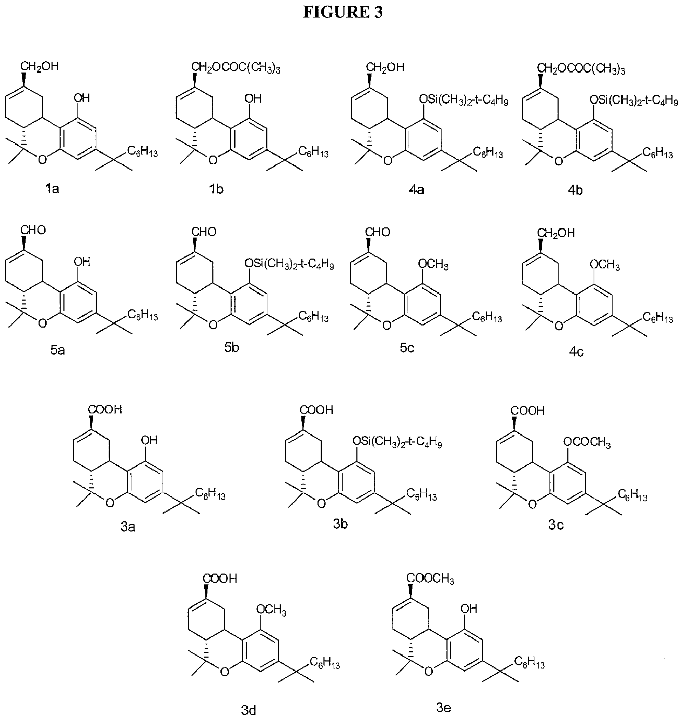

Figure 3 shows several (3R,4R)-Δ8-tetrahydrocannabinol-11-oic acid derivatives.

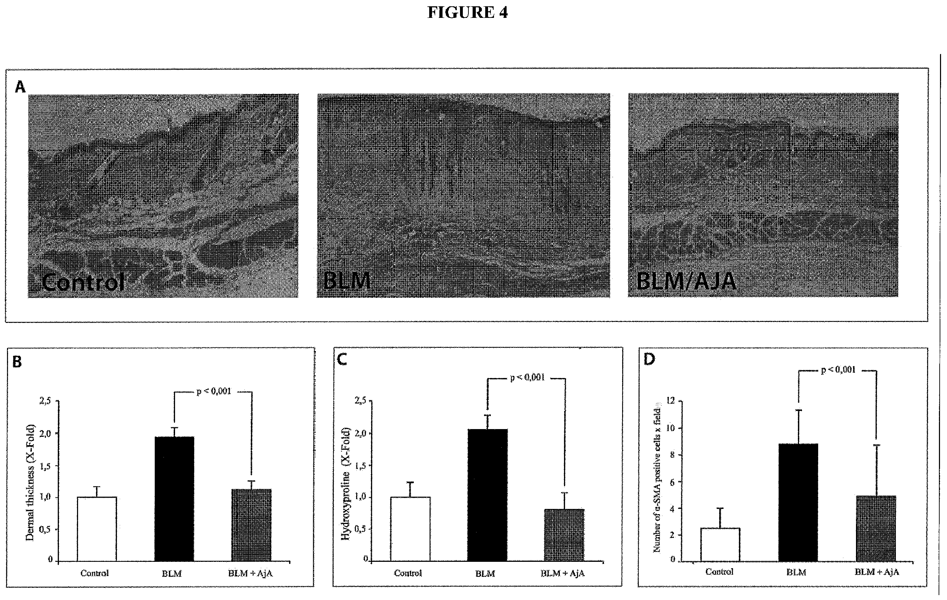

Figure 4 Ajulemic acid (AjA) prevents dermal fibrosisinduced by bleomycin (BLM). (A) Dermal thickening, epidermal hypertrophy, subcutaneous fat atrophy and leucocyte infiltration in the deep dermal layers and perivascular spaces were seen in lesional skin from BLM-challenged mice. Treatment with AjA (1 mg/kg/day) preserved dermal architecture and subcutaneous fat layers, and reduced the leucocyte infi ltration markedly. Haematoxylin and eosin staining. Original magnification × 10. (B) Histograms showing dermal thickness of lesional skin. After 21 days of treatment with AjA (1 mg/kg/day) dermal thickness was equal to that of the control group. (C) Histograms showing hydroxyproline content of lesional skin. After 21 days of treatment with AjA (1 mg/kg/day) dermal thickness was equal to that of the control group. (D) Histograms showing significant reduction in the number of α-smooth muscle actin (a-SMA)-positive spindle-shaped fi broblastic cells per microscopic high field after AjA treatment. Each treatment group, n=8. All values are mean±SD. Analysis of variance was used to compare multiple means, followed by the Student-Newman Keuls post hoc test for multiple comparisons.

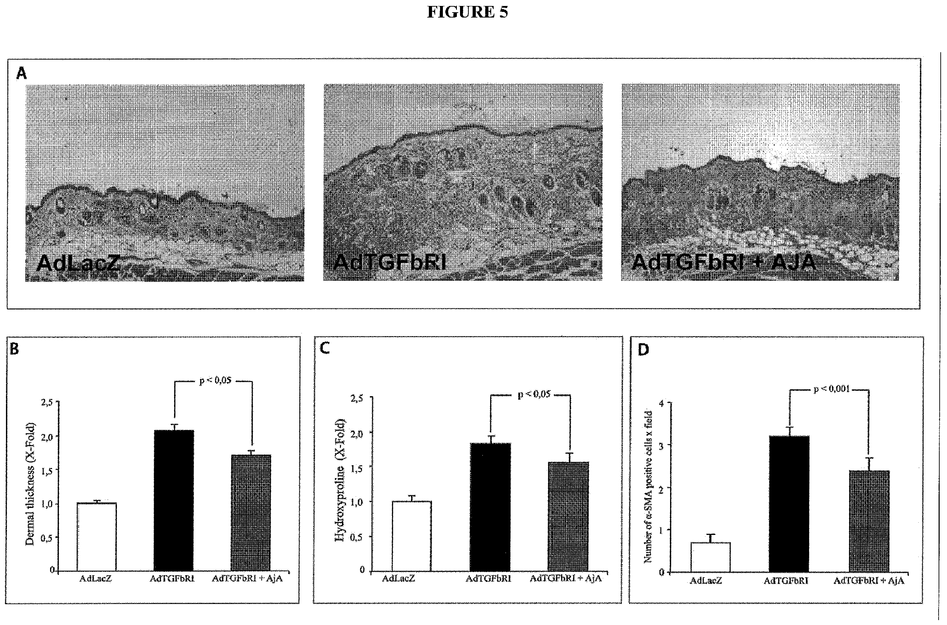

Figure 5 shows antifibrotic effects of ajulemic acid (AjA) in AdTGFbRI mice. (A) Dermal fibrosis in AdTGFbRI mice treated with AjA (1 mg/kg/day) was reduced compared with mock-treated mice. Haematoxylin and eosin staining. Original magnification ×10. (B) Histograms showing a significant reduction in dermal thickness of AdTGFbRI lesional skin after AjA treatment. (C) Hydroxyproline content of AdTGFbRI lesional skin was reduced after AjA treatment. (D) Histograms showing significant reduction in α-smooth muscle actin (a-SMA)-positive spindle-shaped fi broblastic cells per microscopic high field after AjA treatment of AdTGFbRI mice. Each treatment group, n=8. All values are mean±SD. Analysis of variance was used to compare multiple means, followed by the Student-Newman Keuls post hoc test for multiple comparisons.

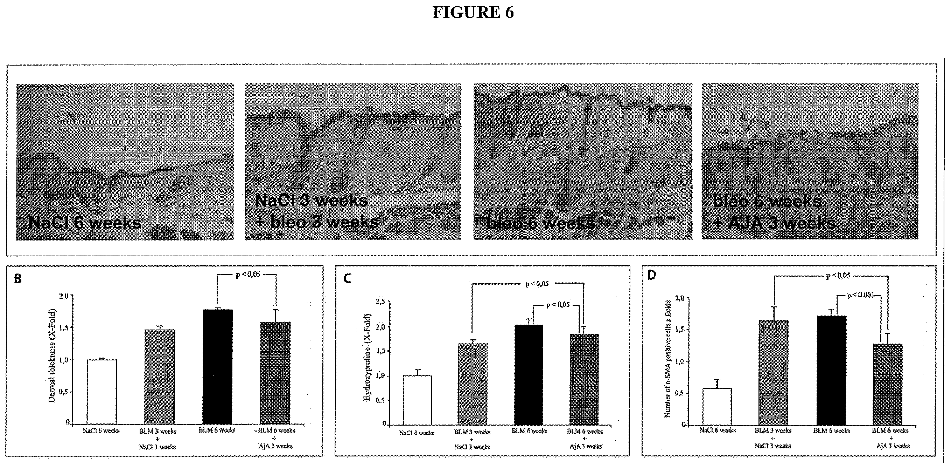

Figure 6 shows Ajulemic acid (AjA) inhibits progression of dermal fibrosisin a modifi ed model of 6 weeks bleomycin (BLM)-induced fibrosis. (A) Treatment with AjA (1 mg/kg/day) prevented further matrix accumulation, but did not induced regression of pre-existing fibrosis. Haematoxylin and eosin staining. Original magnification ×10. (B) Treatment with AjA reduced dermal thickness of lesional skin for the last 3 weeks. (C) Histograms showing hydroxyproline content of lesional skin. AjA treatment disrupted collagen deposition for the last 3 weeks, without regression of pre-existing extracellular matrix accumulation. (D) Histograms showing a signifi cant reduction in the number of α-smooth muscle actin (α-SMA)-positive spindle-shaped fibroblastic cells per microscopic high field after treatment with AjA. Each treatment group, n=8. All values are mean±SD. Analysis of variance was used to compare multiple means, followed by the Student-Newman Keuls post hoc test for multiple comparisons.

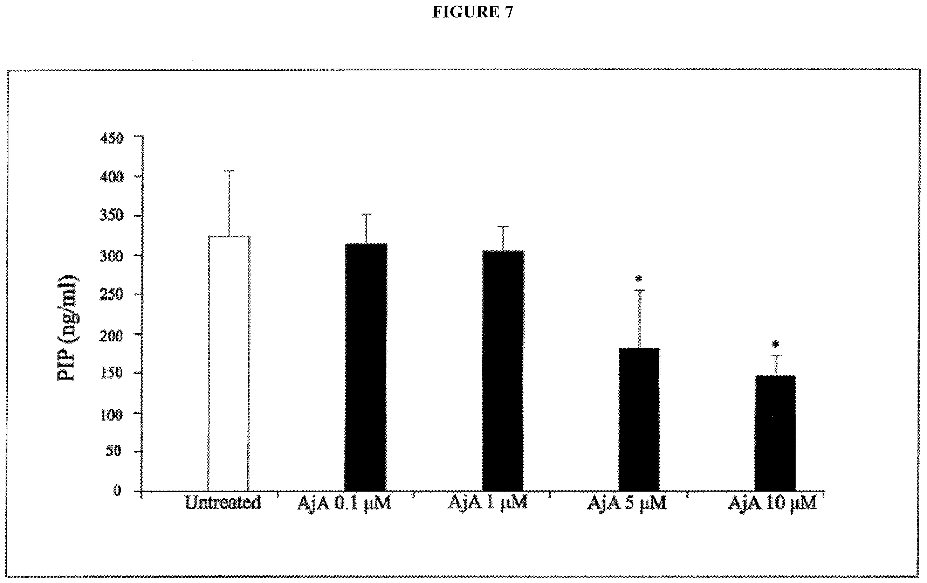

Figure 7 Ajulemic acid (AjA) decreases collagen neosynthesis in diffuse cutaneous systemic sclerosis (dcSSc) fibroblasts in a dosedependent manner. Treatment with AjA (0.1, 1, 5 and 10 µM) induced a dose-dependent reduction of supernatant procollagen type I propeptide (PIP) levels from SSc fibroblasts. Results are the mean±SD of five separate experiments. *p<0.001 versus untreated.

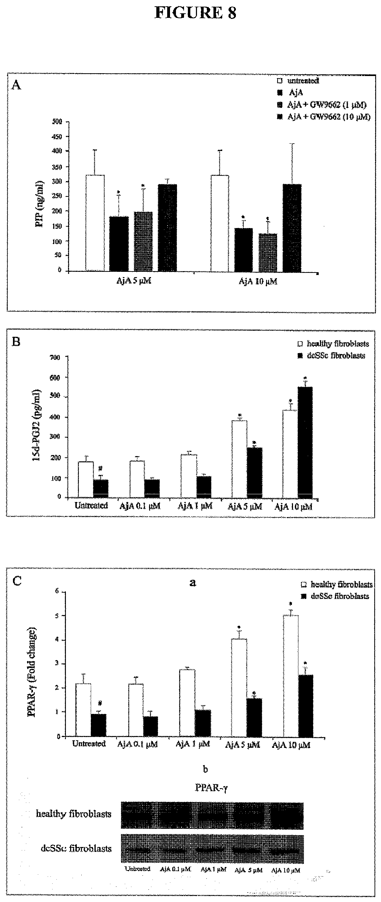

Figure 8 shows the anti-fibrotic effects of ajulemic acid (AjA) are mediated by peroxisome proliferator-activated receptor-y (PPAR-γ). (A) AjA inhibitory effect on procollagen type I propeptide (PIP) production was completely reversed by preincubation of cells with the PPAR-γ irreversible antagonist GW9662 at 10 µM. Results are the mean±SD of five separate experiments. *p<0,001 versus untreated. (B) AjA enhanced production of 15d-PGJ2 in systemic sclerosis (SSc) fibroblasts. Smaller amounts of the natural ligand of PPAR-γ, 15d-PGJ2, were found in the supernatant from diffuse cutaneous SSc (dcSSc) fibroblasts compared with the amounts from healthy controls. After AjA treatment supernatant levels of 15d-PGJ2 increased significantly in a dose-dependent manner. Results are the mean±SD of five separate experiments. #p<0.001 versus healthy fibroblasts; *p<0.001 vs untreated. (C) AjA increased PPAR-γ expression in dcSSc fibroblasts. dcSSc and healthy fibroblasts were treated with AjA at increasing concentrations (0.1, 1, 5 and 10 µM). At 24 h after treatment, cells were lysed and PPAR-γ protein levels were analysed by western blot (a) and densitometry (b). Lower PPAR-γ levels were seen in dcSSc fibroblasts than in healthy controls. After AjA treatment (5 µM and 10 µM) PPAR-γ protein expression increased significantly. Results are the mean±SD of three separate experiments. #p<0.001 versus healthy fibroblasts; *p<0.001 versus untreated.

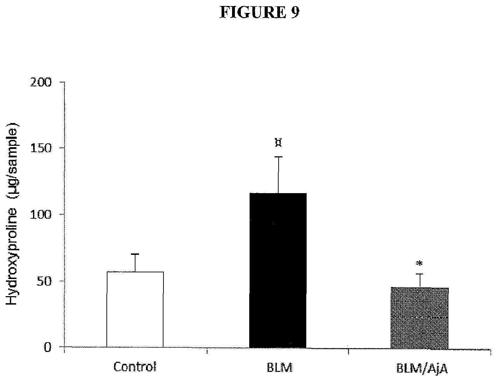

Figure **9** shows histograms showing hydroxyproline content of lesional skin. After 21 days of treatment with AjA (1 mg/kg/day) collagen content as measured by hydroxyproline was equal to control group. (mean±SD) *p<0.001, ANOVA.

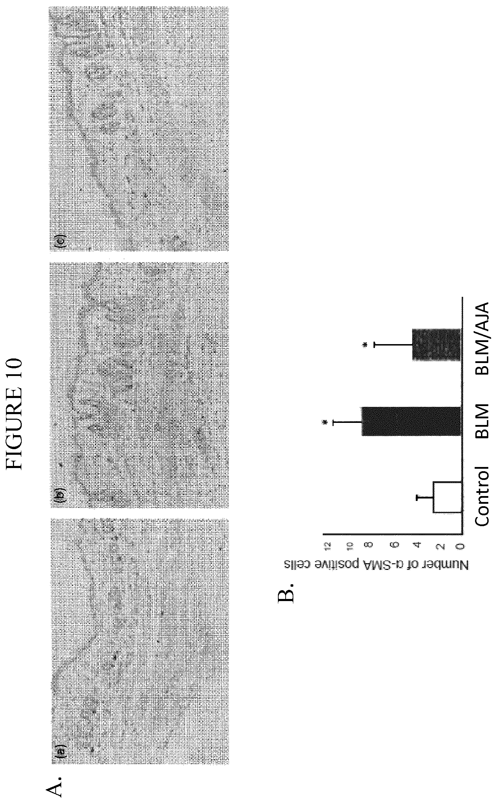

Figure **10** shows AjA inhibited fibroblast activation induced by bleomycin. (A) Immunohistochemistry shows a strong downregulation of alpha-SMA positive cells after AjA (1 mg/kg/day) treatment. Original magnification 10x. (B) alpha-SMA positive spindle-shaped fibroblastic cells per microscopic high-field. (mean±SD) *p<0.001. ANOVA.

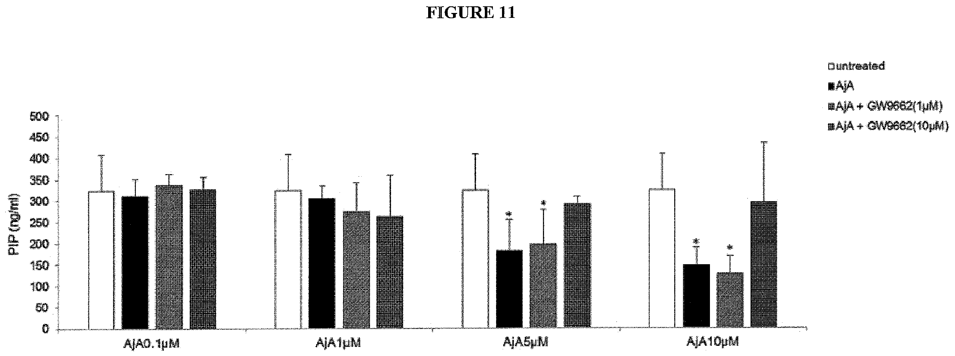

Figure **11** shows AjA inhibited synthesis of collagen in SSc fibroblasts through PPAR-γ. Treatment with AjA (0.1, 1, 5 and 10 µM) induced a dose-dependent reduction of supernatant PIP levels from SSc fibroblasts. AjA inhibitory effect on PIP production was completely reverted by pre-incubation of cells with the PPAR-γ irreversible antagonist GW9662 at 10 µM. (mean±SD) *p<0.001;ANOVA.

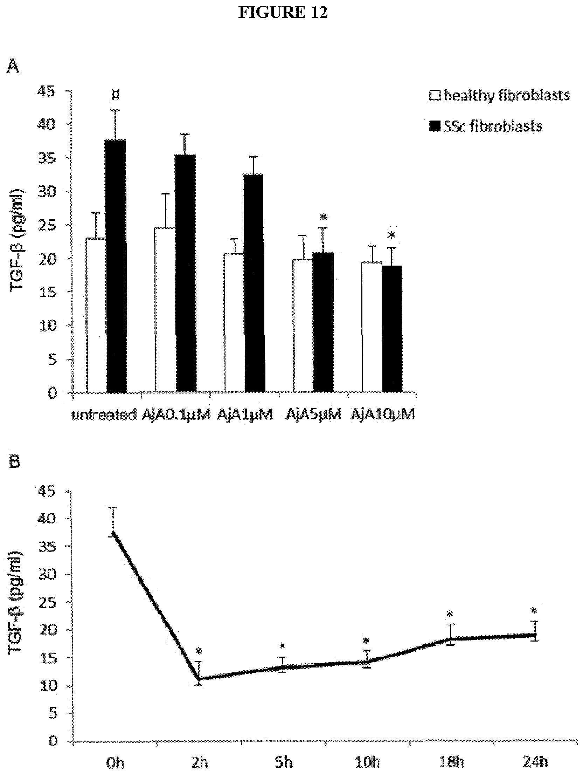

Figure **12** shows AjA downregulated the release of TGF-beta in SSc fibroblasts. (A) AjA (5 and 10 microM) treatment resulted in a significant reduction of supernatant TGF-beta. (B) The maximum of TGF-beta inhibition was observed after 2 hours of AjA treatment (10 microM) and remained significantly downregulated until 24 hours. *p<0.001 vs control fibroblasts; *p<0,001 vs untreated; ANOVA.

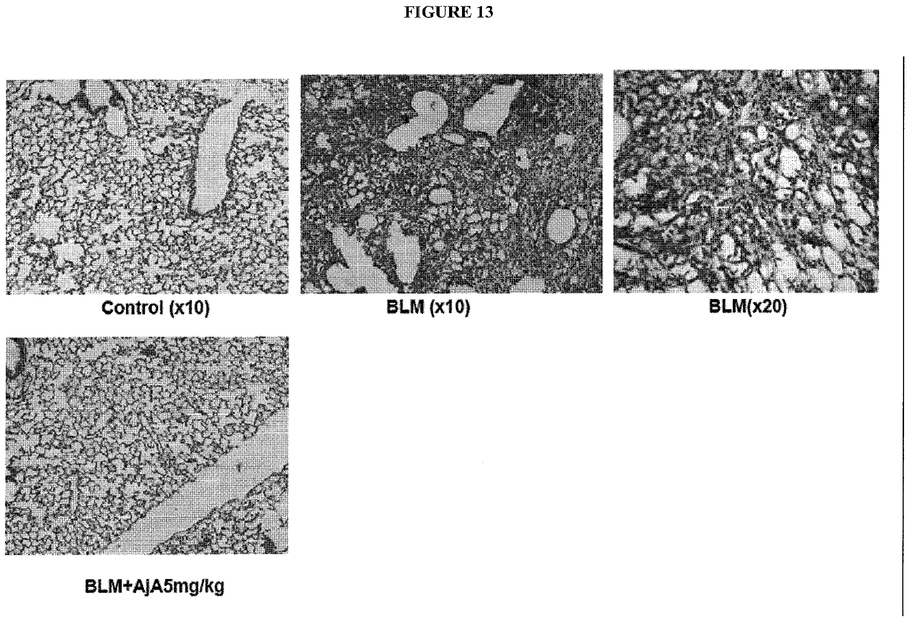

Figure **13** shows Day 21 Histology of Lung of Control (Vehicle), Bleomycin, and Bleomycin + ajulemic acid (5mg/kg qd x 21d). In the BLM group histology revealed strong inflammation with diffuse parenchymal fibrotic areas. After AjA treatment parenchymal infiltration of leukocytes was reduced (more evident in the AjA 5mg/kg group) and fibrosis was significantly inhibited.

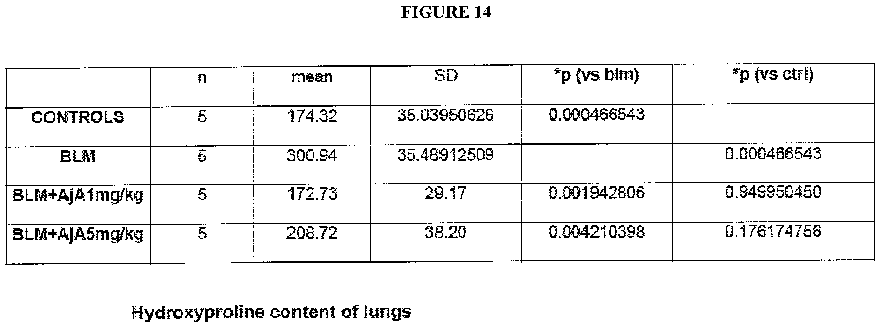

Figure 14 shows Day 21 Hydroxyproline content in lungs of control, bleomycin, and bleomycin + 1 mg/kg and 5 mg/kg ajulemic acid administered qd x 21 days.

### DETAILED DESCRIPTON OF THE INVENTION

### 1. THC DERIVATIVES

Tetrahydrocannabinol (THC) is the major psychoactive constituent of marijuana. In addition to mood-altering effects, THC has been reported to exhibit other activities, some of which may have therapeutic value, including analgesic, anti-inflammatory and anti-emetic properties. The potential therapeutic value of THC has led to a search for related compounds which minimize the psychoactive effects, while retaining the activities of potential medicinal value.

For example, (6aR,10aR)-4-(1,1-dimethylheptyl)-Δ8-tetrahydro-cannabinol-9-carboxylic acid, also known as ajulemic acid ((6aR,10aR)-3-(1,1-dimethylheptyl)-6a,7,10,10a-tetrahydro-1-hydroxy-6,6-dimethyl-6H-Dibenz o(b,d)pyran-9-carboxylic acid IUPAC name), is a candidate for the treatment of pain and inflammation either alone or in combination with other agents. This compound is a mixed CB1- and CB2-agonist, which has shown clinical evidence of efficacy and safety in normal healthy volunteers and patients with refractory, traumatic neuropathic pain. The current body of knowledge of cannabinoid research in pain and inflammation suggests that CB1 and CB2 receptors play an important role in the initiation and maintenance of post-synaptic signalling and immune mechanisms related to nociception, sensitization, pain signal transmission and pain processing. (6aR,10aR)-4-(1,1-dimethylheptyl)-Δ8-tetrahydro-cannabinol-9-carboxylic acid has a high affinity for both CB1 and CB2 receptors.

### 2. FIBROTIC DISEASE

Fibrosis is the abnormal accumulation of fibrous tissue that can occur as a part of the wound-healing process in damaged tissue. Examples of fibrosis include liver fibrosis, lung fibrosis (e.g., silicosis, asbestosis, idiopathic pulmonary fibrosis), oral fibrosis, endomyocardial fibrosis, retroperitoneal fibrosis, deltoid fibrosis, kidney fibrosis (including diabetic nephropathy), and glomerulosclerosis. Liver fibrosis, for example, occurs as a part of the wound-healing response to chronic liver injury. Fibrosis can occur as a complication of haemochromatosis, Wilson's disease, alcoholism, schistosomiasis, viral hepatitis, bile duct obstruction, exposure to toxins, and metabolic disorders. The formation of fibrotic tissue is believed to represent an attempt by the body to encapsulate injured tissue. Liver fibrosis is characterized by the accumulation of extracellular matrix that can be distinguished qualitatively from that in normal liver. Left unchecked, hepatic fibrosis progresses to cirrhosis (defined by the presence of encapsulated nodules), liver failure, and death. Endomyocardial fibrosis is an idiopathic disorder that is characterized by the development of restrictive cardiomyopathy. In endomyocardial fibrosis, the underlying process produces patchy fibrosis of the endocardial surface of the heart, leading to reduced compliance and, ultimately, restrictive physiology as the endomyocardial surface becomes more generally involved. Endocardial fibrosis principally involves the inflow tracts of the right and left ventricles and may affect the atrioventricular valves, leading to tricuspid and mitral regurgitation. Oral submucous fibrosis is a chronic, debilitating disease of the oral cavity characterized by inflammation and progressive fibrosis of the submucosal tissues (lamina propria and deeper connective tissues). It results in marked rigidity and an eventual inability to open the mouth. The buccal mucosa is the most commonly involved site, but any part of the oral cavity can be involved, even the pharynx. Retroperitoneal fibrosis is characterized by the development of extensive fibrosis throughout the retroperitoneum, typically centered over the anterior surface of the fourth and fifth lumbar vertebrae. This fibrosis leads to entrapment and obstruction of retroperitoneal structures, notably the ureters. In most cases, the etiology is unknown.

Scleroderma is a fibrotic disease that affects approximately 19 cases per 1 million persons. The cause of scleroderma is unknown. Abnormalities involve autoimmunity and alteration of endothelial cell and fibroblast function are believed to be involved. Indeed, systemic sclerosis is probably the most severe of the auto-immune diseases with 50% mortality within 5 years of diagnosis.

Scleroderma is a disease of the connective tissue characterized by fibrosis of the skin and internal organs, leading to organ failure and death. Scleroderma has a spectrum of manifestations and a variety of therapeutic implications. It comprises localized scleroderma, systemic sclerosis, scleroderma-like disorders, and sine scleroderma.

Whilst localized scleroderma is a rare dermatologic disease associated with fibrosis and manifestations limited to skin, systemic sclerosis is a multi-system disease with variable risk for internal organ involvement and variation in the extent of skin disease. Systemic sclerosis can be diffuse or limited. Limited systemic sclerosis is also called CREST (calcinosis, Raynaud's esophageal dysfunction, sclerodactyly, telangiectasiae). Systemic sclerosis comprises: scleroderma lung disease, scleroderma renal crisis, cardiac manifestations, muscular weakness including fatigue or limited CREST, gastrointestinal dysmotility and spasm, and abnormalities in the central, peripheral and autonomic nervous system. Scieroderma-like disorders are believed to be related to industrial environment exposure. In sine disease, there is internal organ involvement without skin changes.

The major symptoms or manifestations of scleroderma and in particular of systemic sclerosis are inappropriate excessive collagen synthesis and deposition, endothelial dysfunction, spasm, collapse and obliteration by fibrosis. In terms of diagnosis, an important clinical parameter is skin thickening proximal to the metacarpophalangeal joints. Raynaud's phenomenon is a frequent, almost universal component of scleroderma. It is diagnosed by color changes of the skin upon cold exposure. Ischemia and skin thickening are symptoms of Raynaud's disease.

Several underlying biological processes are implicated in the initiation, severity and progression of the disease and include vascular dysfunction, endothelial cell activation and damage, leukocyte accumulation, auto-antibody production and crucially, an uncontrolled fibrotic response which may lead to death. Fibroblasts have a pivotal role in the pathogenesis of this disease. Primary fibroblasts obtained from patients with scleroderma exhibit many of the characteristic properties of the disease seen in vivo, notably increased extracellular matrix synthesis and deposition, notably of collagen and fibronectin, and altered growth factor and cytokine production such as of TGF-beta and CTGF ("Increased collagen synthesis by scleroderma skin fibroblasts in vitro" J. Clin. Invest. 54, p. 880-89 LeRoy (1974)) [2].

There is no curative treatment of scleroderma. Innovative but high-risk therapy proposed autologous stem cell transplantation. In particular, there are currently no treatments for scleroderma targeting the fibrotic process. Identification of the genes associated with disease risk and scleroderma progression may lead to the development of effective strategies for intervention at various stages of the disease.Although there is presently no cure for scleroderma, several agents or treatments are presently being used to treat scleroderma symptoms.

Applicants have discovered that compounds having the formula 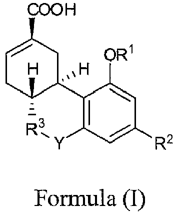 wherein R₁ is hydrogen, COCH₃ or COCH₂CH₃; R₂ is a branched C5-C12 alkyl group which may optionally have a terminal aromatic ring, or optionally a branched OCHCH₃(CH₂)ₘ alkyl group which may have a terminal aromatic ring, wherein m is 0 to 7; R₃ is hydrogen, a C1-8 alkyl or a Cl-8 alkanol group; and Y is nil or a bridging group of NH or oxygen, provided that where Y is oxygen and R₂ is a branched C5-C12 alkyl, R₃ is not CHCH₃, and pharmaceutically acceptable salts, esters, or solvate thereof, can be used for the treatment and/or prevention of fibrotic diseases.

In particular, Applicants have discovered that administration of (3R,4R)-Δ8-tetrahydrocannabinol-11-oic acids such as (6aR,10aR)-4-(1,1-dimethylheptyl)-Δ8-tetrahydro-cannabinol-9-carboxylic acid, also known as ajulemic acid, is effective in treating tissue fibrosis of the lung and skin, as demonstrated using a well-established animal model of scieroderma.

In accordance with the present invention, ajulemic acid and other (3R,4R)-Δ8-tetrahydrocannabinol-ll-oic acids, or compositions containing these compounds, can be used, in the treatment and/or prevention of various fibrotic diseases, including scleroderma, systemic sclerosis, scleroderma-like disorders, sine scleroderma, liver cirrhosis, interstitial pulmonary fibrosis, idiopathic pulmonary fibrosis, Dupuytren's contracture, keloids, chronic kidney disease, and other scarring/wound healing abnormalities, post operative adhesions, and reactive fibrosis.

Various dosage forms of ajulemic acid and other (3R,4R)-Δ8-tetrahydrocannabinol-11-oic acids can be used in the methods of the present invention for preventing and/or treating fibrotic conditions. In certain embodiments, the dosage form is an oral dosage form such as a tablet or capsule or enteric coated tablet or osmotic release capsule or unique combination of excipients formulated in such a way as to deliver over a 24 hour period not more than 240mg and not less than 5mg of ajulemic acid or other (3R,4R)-Δ8-tetrahydrocannabinol-11-oic acids, more preferably not more than 180 mg and not less than 15mg, (e.g., from about 120mg to about 30mg). In other embodiments, the dosage form is a topical patch, gel, ointment, cream, aerosol, or inhaled formulation.

In further embodiments, the dosage form includes an additional agent or is provided together with a second dosage form, which includes the additional agent. Exemplary additional agents include an analgesic agent such as an NSAID or opiate, or an anti-inflammatory agent. In additional embodiments, the dosage form comprises a capsule wherein the capsule contains a mixture of materials to provide a desired sustained release formulation.

In other embodiments, the dosage form comprises a tablet coated with a semipermeable coating. In certain embodiments, the tablet comprises two layers, a layer containing ajulemic acid or another (3R,4R)-Δ8-tetrahydrocannabinol-11-oic acid and a second layer referred to as a "push" layer. The semi-permeable coating is used to allow a fluid (e.g., water) to enter the tablet and erode a layer or layers. In certain embodiments, this sustained release dosage form further comprises a laser hole drilled in the center of the coated tablet. The ajulemic acid or other (3R,4R)-Δ8-tetrahydrocannabinol-11-oic acid containing layer comprises ajulemic acid or another (3R,4R)-Δ8-tetrahydrocannabinol-11-oic acid, a disintegrant, a viscosity enhancing agent, a binding agent and an osmotic agent. The push layer comprises a disintegrant, a binding agent, an osmotic agent and a viscosity enhancing agent.

In another aspect, the invention features a dosage form of (6a R,10aR)-4-(1,1-dimethylheptyl)-Δ8-tetrahydro-cannabinol-9-carboxylic acid or another (3R,4R)-Δ8-tetrahydrocannabinol-11-oic acid that is a controlled release dosage form, which provides controlled release of (6aR,10aR)-4-(1,1-dimethylheptyl)-Δ8-tetrahydro-cannabinol-9-carboxylic acid or the other (3R,4R)-Δ8-tetrahydrocannabinol-11-oic acid.

In further embodiments, the dosage form comprises a tablet comprising a biocompatible matrix and ajulemic acid or another (3R,4R)-Δ8-tetrahydrocannabinol-11-oic acid. The sustained release dosage form may also comprise a hard-shell capsule containing bio-polymer microspheres that contains the therapeutically active agent. The biocompatible matrix and bio-polymer microspheres each contain pores for drug release and delivery. These pores are formed by mixing the biocompatible matrix of bio-polymer microsphere with a pore forming agent. Each biocompatible matrix or bio-polymer microsphere is made up of a biocompatible polymer or mixture of biocompatible polymers. The matrix and microspheres can be formed by dissolving the biocompatible polymer and active agent (compound described herein) in a solvent and adding a pore forming agent (e.g., a volatile salt). Evaporation of the solvent and pore forming agent provides a matrix or microsphere containing the active compound. In additional embodiments, the sustained release dosage form comprises a tablet, wherein the tablet contains ajulemic acid or another (3R,4R)-Δ8-tetrahydrocannabinol-11-oic acid and one or more polymers and wherein the tablet can be prepared by compressing the ajulemic acid or other (3R,4R)-Δ8-tetrahydrocannabinol-11-oic acid and one or more polymers. In some embodiments, the one or more polymers may comprise a hygroscopic polymer formulated with ajulemic acid or other (3R,4R)-Δ8-tetrahydrocannabinol-11-oic acid. Upon exposure to moisture, the tablet dissolves and swells. This swelling allows the sustained release dosage form to remain in the upper GI tract. The swelling rate of the polymer mixture can be varied using different grades of polyethylene oxide.

In other embodiments, the sustained release dosage form comprises a capsule further comprising particle cores coated with a suspension of active agent and a binding agent which is subsequently coated with a polymer. The polymer may be a rate-controlling polymer. In general, the delivery rate of the rate-controlling polymer is determined by the rate at which the active agent is dissolved.

### 3. PREFERRED EMBODIMENTS

As noted above, Applicants have discovered that administration of (3R,4R)-Δ8-tetrahydrocannabinol-11-oic acids such as (6aR,10aR)-4-(1,1-dimethylheptyl)-Δ8-etrahydro-cannabinol-9-carboxylic acid, also known as ajulemic acid, can be used to treat or prevent fibrotic diseases in a subject.

### A. SYNTHESIS OF (3R,4R)-Δ8-TETRAHYDROCANNABINOL-11-OIC ACIDS

In one embodiment, (3R,4R)-Δ8-tetrahydrocannabinol-11-oic acids such as (6aR,10aR)-4-(1,1- dimethylheptyl)-Δ8-tetrahydro-cannabinol-9-carboxylic acid is synthesized according to the scheme in Figure 2.

### B. UNIT DOSAGE FORMULATIONS

Various dosage forms of (6aR,10aR)-4-(1,1-dimethymeptyl)-Δ8-tetrahydrocannabinol-9-carboxylic acid and other (3R,4R)-Δ8-tetrabydrocannabinol-11-oic acids can be administered to a subject for the treatment and/or prevention of fibrotic diseases in the subject. Exemplary dosage forms include oral dosage forms (e.g., a tablet or capsule), topical dosage forms such as a topical patch, gels, and ointments, and inhaled dosage forms such as inhalers, nebulizers, aerosols and sprays.

In certain embodiments, the (6aR,10aR)-4-(1,1-dimethylheptyl)-Δ8-tetrahydro-cannabinol-9-carboxylic acid or other (3R,4R)-Δ8-tetrahydrocannabinol-11-oic acid is formulated into a dosage form wherein a single dosage is from about 5mg to about 120 mg once daily or from about 2 mg to about 40 mg up to 3 times daily.

In other embodiments, the (6aR,10aR)-4-(1,1-dimethylheptyl)-Δ8-tetrahydro-cannabinol-9-carboxylic acid or other (3R,4R)-Δ8-tetrahydrocannabinol-11-oic acid is formulated into a dosage form wherein a single dosage is from about 0.1 to about 0.8 mg/kg weight of the subject. In further embodiments, the dosage form is administered up to 3 times daily and from about 0.3 to about 2.4 mg/kg weight of the subject once daily.

### C. FORMULATIONS

In some embodiments, one or more of the therapeutic agents that can be used in the methods of the present invention for preventing and/or treating fibrotic conditions are formulated with a pharmaceutically acceptable carrier, vehicle or adjuvant. The term "pharmaceutically acceptable carrier, vehicle or adjuvant" refers to a carrier, vehicle or adjuvant that may be administered to a patient, together with a compound that can be used in the methods of the present invention for preventing and/or treating fibrotic conditions, and which does not destroy the pharmacological activity thereof and is nontoxic when administered in doses sufficient to deliver a therapeutic amount of the compound.

Pharmaceutically acceptable carriers, adjuvants and vehicles that may be used in the dosage forms of this invention include, but are not limited to, ion exchangers, alumina, aluminum stearate, lecithin, self-emulsifying drug delivery systems (SEDDS) such as d-E-tocopherol polyethyleneglycol 1000 succinate; surfactants used in pharmaceutical dosage forms such as Tweens or other similar polymeric delivery matrices; serum proteins such as human serum albumin; buffer substances such as phosphates, glycine, sorbic acid, potassium sorbate, partial glyceride mixtures of saturated vegetable fatty acids, water, salts; or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxmethylcellulose, polyacrylates, waxes, polyethylene-polyoxypropylene-block polymers, polyethylene glycol and wool fat. Cyclodextrins such as alpha, beta and γ-cyclodextrin, or chemically modified derivatives such as hydroxyalkylcyclodextrins, including 2- and 3-hydroxypropyl-beta cyclodextrins, or other solubilized derivatives may also be advantageously used to enhance delivery of compounds of the formulae described herein that can be used in the methods of the present invention for preventing and/or treating fibrotic conditions. Additional suitable excepients may be found in Handbook of Pharmaceutical Exceptients, R. C. Rowe, et.al., Pharmaceutical Press, 2009. In certain embodiments, unit dosage formulations are compounded for immediate release, though unit dosage formulations compounded for delayed or prolonged release of one or both agents are also disclosed.

In some embodiments, the therapeutic agents that can be used in the methods of the present invention for preventing and/or treating fibrotic conditions are formulated in a single unit dose such that the agents are released from the dosage at different times.

In some embodiments, for example where one or more of the therapeutic agents is administered once or twice per day, the agent is formulated to provide extended release. For example, the agent is formulated with an enteric coating. In an alternative embodiment, the agent is formulated using a biphasic controlled release delivery system, thereby providing prolonged gastric residence. For example, in some embodiments, the delivery system includes (1) an inner solid particulate phase formed of substantially uniform granules containing a pharmaceutical having a high water solubility, and one or more hydrophilic polymers, one or more hydrophobic polymers and/or one or more hydrophobic materials such as one or more waxes, fatty alcohols and/or fatty acid esters, and (2) an outer solid continuous phase in which the above granules of inner solid particulate phase are embedded and dispersed throughout, the outer solid continuous phase including one or more hydrophobic polymers, one or more hydrophobic polymers and/or one or more hydrophobic materials such as one or more waxes, fatty alcohols and/or fatty acid esters, which may be compressed into tablets or filled into capsules. In some embodiments, the agent is incorporated into polymeric matrices comprised of hydrophilic polymers that swell upon imbibition of water to a size that is large enough to promote retention of the dosage form in the stomach during the fed mode.

In some embodiments, the (6aR,10aR)-4-(1,1-dimethylheptyl)-Δ8-tetrahydro-cannabinol-9-carboxylic acid, in the formulation is formulated as a combination of fast-acting and controlled release forms.

In some embodiments, the (6aR,10aR).-4-(1,1-dimethylheptyl)-Δ8-tetrahydrocannabinol-9-carboxylic acid is formulated with a single release property. For example, it is not present in a modified release form, e.g., a controlled release form.

Compositions disclosed herein that can be used in the methods of the present invention for preventing and/or treating fibrotic conditions can be taken just prior to or with each of three meals, each of two major meals, or one meal. In other embodiments, a composition disclosed herein can be administered once a day or twice a day and need not be administered just before or with a meal.

The dosage forms of this invention that can be used in the methods of the present invention for preventing and/or treating fibrotic conditions may be administered orally, parentrally, by inhalation spray, topically, rectally, nasally, buccally, vaginally or via an implanted reservoir, preferably by oral administration or administration by injection. The pharmaceutical compositions of this invention that can be used in the methods of the present invention for preventing and/or treating fibrotic conditions may contain any conventional non-toxic pharmaceutically-acceptable carriers, adjuvants or vehicles. In some cases, the pH of the formulation may be adjusted with pharmaceutically acceptable acids, bases or buffers to enhance the stability of the formulated compound or its delivery form. The term parenteral as used herein includes subcutaneous, intracutaneous, intravenous, intramuscular, intraarticular, intraarterial, intrasynovial, intrasternal, intrathecal, intralesional and intracranial injection or infusion techniques.

The dosage forms that can be used in the methods of the present invention for preventing and/or treating fibrotic conditions may be in the form of a sterile injectable preparation, for example, as a sterile injectable aqueous or oleaginous suspension. This suspension may be formulated according to techniques known in the art using suitable dispersing or wetting agents (such as, for example, Tween 80) and suspending agents. The sterile injectable preparation may also be a sterile injectable solution or suspension in a nontoxic parenterally acceptable diluent or solvent, for example, as a solution in 1,3-butanediol. Among the acceptable vehicles and solvents that may be employed are mannitol, water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, or carboxymethyl cellulose or similar dispersing agents which are commonly used in the formulation of pharmaceutically acceptable dosage forms such as emulsions and or suspensions. Other commonly used surfactants such as Tweens or Spans and/or other similar emulsifying agents or bioavailability enhancers which are commonly used in the manufacture of pharmaceutically acceptable solid, liquid, or other dosage forms may also be used for the purposes of formulation.

The agents described herein that can be used in the methods of the present invention for preventing and/or treating fibrotic conditions are preferably administered orally, for example as a component in a dosage form. The dosage forms may contain any conventional non-toxic pharmaceutically-acceptable carriers, adjuvants or vehicles. In some cases, the pH of the formulation may be adjusted with pharmaceutically acceptable acids, bases or buffers to enhance the stability of the formulated compound or its delivery form.

The dosage forms of this invention may be orally administered in any orally acceptable dosage form including, but not limited to, capsules, tablets, emulsions and aqueous suspensions, dispersions and solutions. In the case of tablets for oral use, carriers that are commonly used include lactose and corn starch. Lubricating agents, such as magnesium stearate, are also typically added. For oral administration in a capsule form, useful diluents include lactose and dried corn starch. When aqueous suspensions and/or emulsions are administered orally, the active ingredient may be suspended or dissolved in an oily phase is combined with emulsifying and/or suspending agents. If desired, certain sweetening and/or flavoring and/or coloring agents may be added.

The dosage forms of this invention that can be used in the methods of the present invention for preventing and/or treating fibrotic conditions may also be administered in the form of suppositories for rectal administration. These compositions can be prepared by mixing a compound of this invention that can be used in the methods of the present invention for preventing and/or treating fibrotic conditions with a suitable non-irritating excipient which is solid at room temperature but liquid at the rectal temperature and therefore will melt in the rectum to release the active components. Such materials include, but are not limited to, cocoa butter, beeswax and polyethylene glycols.

Topical administration of the dosage forms of this invention that can be used in the methods of the present invention for preventing and/or treating fibrotic conditions is useful when the desired treatment involves areas or organs readily accessible by topical application. For application topically to the skin, the dosage form should be formulated with a suitable ointment containing the active components suspended or dissolved in a carrier. Carriers for topical administration of the compounds of this invention that can be used in the methods of the present invention for preventing and/or treating fibrotic conditions include, but are not limited to, mineral oil, liquid petroleum, white petroleum, propylene glycol, polyoxyethylene polyoxypropylene compound, emulsifying wax and water. Alternatively, the pharmaceutical composition that can be used in the methods of the present invention for preventing and/or treating fibrotic conditions can be formulated with a suitable lotion or cream containing the active compound suspended or dissolved in a carrier with suitable emulsifying agents. Suitable carriers include, but are not limited to, mineral oil, sorbitan monostearate, polysorbate 60, cetyl esters wax, cetearyl alcohol, 2-octyldodecanol, benzyl alcohol and water. The pharmaceutical compositions of this invention that can be used in the methods of the present invention for preventing and/or treating fibrotic conditions may also be topically applied to the lower intestinal tract by rectal suppository formulation or in a suitable enema formulation. Topically-transdermal patches are also included in this invention.

The dosage forms of this invention that can be used in the methods of the present invention for preventing and/or treating fibrotic conditions may be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art.

When the dosage forms of this invention that can be used in the methods of the present invention for preventing and/or treating fibrotic conditions comprise a combination of a compound of the formulae described herein and one or more additional therapeutic or prophylactic agents, both the compound and the additional agent should be present at dosage levels of between about 1 to 100%, and more preferably between about 5 to 95% of the dosage normally administered in a monotherapy regimen. The additional agents may be administered separately, as part of a multiple dose regimen, from the compounds of this invention. Alternatively, those agents may be part of a single dosage form, mixed together with the compounds of this invention in a single composition.

In certain embodiments, the dosage form that can be used in the methods of the present invention for preventing and/or treating fibrotic conditions comprises a capsule wherein the capsule comprises a mixture of material to provide the desired sustained release.

In other embodiments, the dosage form that can be used in the methods of the present invention for preventing and/or treating fibrotic conditions comprises a tablet coated with a semi-permeable coating. In certain embodiments, the tablet comprises two layers, a layer containing ajulemic acid or other (3R,4R)-Δ8-tetrahydrocannabinol-11-oic acid and a second layer referred to as a "push" layer. The semi-permeable coating is used to allow a fluid (e.g., water) to enter the tablet and erode a layer or layers. In certain embodiments, the sustained release dosage form further comprises a laser hole drilled in the center of the coated tablet. The ajulemic acid or other (3R,4R)-Δ8-tetrahydrocannabinol-11-oic acid containing layer comprises ajulemic acid or another (3R,4R)-Δ8-tetrahydrocannabinol-11-oic acid, a disintegrant, a viscosity enhancing agent, a binding agent and an osmotic agent. The push layer comprises a disintegrant, a binding agent, an osmotic agent and a viscosity-enhancing agent.

In further embodiments, the dosage form that can be used in the methods of the present invention for preventing and/or treating fibrotic conditions comprises a tablet comprising a biocompatible matrix and an ajulemic acid or another (3R,4R)-Δ8-tetrahydrocannabinol-11-oic acid. The sustained release dosage form may also comprise a hard-shell capsule containing bio-polymer microspheres that contains the therapeutically active agent. The biocompatible matrix and bio-polymer microspheres each contain pores for drug release and delivery. These pores are formed by mixing the biocompatible matrix or bio-polymer microsphere with a pore forming agent. Each biocompatible matrix of bio-polymer microsphere is made up of a biocompatible polymer or mixture of biocompatible polymers. The matrix and microspheres can be formed by dissolving the biocompatible polymer and active agent (compound described herein) in a solvent and adding a pore forming agent (e.g., a volatile salt). Evaporation of the solvent and pore forming agent provides a matrix or microsphere containing the active compound.

In additional embodiments, the sustained release dosage form that can be used in the methods of the present invention for preventing and/or treating fibrotic conditions comprises a tablet, wherein the tablet contains ajulemic acid or another (3R,4R)-Δ8-tetrahydrocannabinol-11-oic acid and one or more polymers and wherein the tablet can be prepared by compressing the ajulemic acid or other (3R,4R)-Δ8-tetrahydrocannabinol-11-oic acid and one or more polymers. In some embodiments, the one or more polymers may comprise a hygroscopic polymer formulated with the ajulemic acid or other (3R,4R)-Δ8-tetrahydrocannabinol-11-oic acid active agent (i.e., a compound described herein). Upon exposure to moisture, the tablet dissolves and swells. This swelling allows the sustained release dosage form to remain in the upper GI tract. The swelling rate of the polymer mixture can be varied using different grades of polyethylene oxide.

In other embodiments, the sustained release dosage form that can be used in the methods of the present invention for preventing and/or treating fibrotic conditions comprises a capsule further comprising particle cores coated with a suspension of active agent and a binding agent which is subsequently coated with a polymer. The polymer may be a rate-controlling polymer. In general, the delivery rate of the rate-controlling polymer is determined by the rate at which the active agent is dissolved.

Examples of capsules include but are not limited to gelatin capsules, HPMC, hard shell, soft shell, or any other suitable capsule for holding a sustained release mixture.

The solvents used in the above sustained release dosage forms include, but are not limited to ethyl acetate, triacetin, dimethyl sulfoxide (DIV1S0), propylene carbonate, N-methylpyrrolidone (NMP), ethyl alcohol, benzyl alcohol, glycofurol, alpha-tocopherol, Miglyol 810, isopropyl alcohol, diethyl phthalate, polyethylene glycol 400 (PEG 400), triethyl citrate, and benzyl benzoate.

The viscosity modifiers used in the above sustained release dosage forms include, but are not limited to caprylic/capric triglyceride (Migliol 810), isopropyl myristate (IPM), ethyl oleate, triethyl citrate, dimethyl phthalate, benzyl benzoate and various grades of polyethylene oxide. The high viscosity liquid carrier used in the above sustained release dosage forms include, but are not limited to sucrose acetate isobutyrate (SAIB) and cellulose acetate butyrate (CAB) 381-20.

Examples of materials that make up preferred semi-permeable layers include, but are not limited to cellulosic polymers such as cellulose acetate, cellulose acylate, cellulose diacylate, cellulose triacylate, cellulose diacetate, cellulose triacetate or any mixtures thereof; ethylene vinyl acetate copolymers, polyethylene, copolymers of ethylene, polyolefins including ethylene oxide copolymers (e.g., Engage® -- Dupont Dow Elastomers), polyamides, cellulosic materials, polyurethanes, polyether blocked amides, and copolymers (e.g., PEBAX®, cellulosic acetate butyrate and polyvinyl acetate). Examples of disintegrants that may be employed in the above sustained release dosage forms include but are not limited to croscarmellose sodium, crospovidone, sodium alginate or similar excipients.

Examples of binding agents that may be employed in the above sustained release dosage forms include but are not limited to hydroxyalkylcellulose, a hydroxyalkylalkylcellulose, or a polyvinylpyrrolidone.

Examples of osmotic agents that may be employed in the above sustained release dosage forms include but are not limited to sorbitol, mannitol, sodium chloride, or other salts. Examples of biocompatible polymers employed in the above sustained release dosage forms include but are not limited to poly(hydroxyl acids), polyanhydrides, polyorthoesters, polyamides, polycarbonates, polyelkylenes, polyelkylene glycols, polyalkylene oxides, polyalkylene terepthalates, polyvinyl alcohols, polyvinyl ethers, polyvinyl esters, polyvinyl halides, polyvinylpyrrolidone, polysiloxanes, poly(vinyl alcohols), poly (vinyl acetate), polystyrene, polyurethanes and co-polymers thereof, synthetic celluloses, polyacrylic acids, poly(butyric acid), poly(valeric acid), and poly(lactide-co-caprolactone), ethylene vinyl acetate, copolymers and blends thereof.

Examples of hygroscopic polymers that may be employed in the above sustained release dosage forms include but are not limited to polyethylene oxide (e.g., polyox® with MWs from 4,000,000 to 10,000,000), cellulose hydroxymethyl cellulose, hydroxyethyl-cellulose, crosslinked polyacrylic acids and xanthum gum.

Examples of rate-controlling polymers the may be employed in the above sustained release dosage forms includes but is not limited to polymeric acrylate, methacrylatelacquer or mixtures thereof, polymeric acrylate lacquer, methacrylate lacquer, an acrylic resin comprising a copolymer of acrylic and methacrylic acid esters or an ammonium methacrylate lacquer with a plasticizer.

### D. METHODS OF TREATMENT

The compounds and compositions described herein can be administered to cells in culture, e.g. in vitro or ex vivo, or to a subject, e.g., in vivo, to treat, prevent, and/or diagnose a variety of fibrotic diseases, including those described herein below.

As used herein, the term "treat" or "treatment" is defined as the administration of a compound, e.g., by any route, e.g., orally, to a subject. The compound can be administered alone or in combination with, a second compound. The subject, e.g., a patient, can be one having a disorder (e.g., a disorder as described herein), a symptom of a disorder, or a predisposition toward a disorder. Treatment can result in one or more of curing, healing, alleviating, relieving, altering, remedying, ameliorating, improving or affecting the disorder, one or more symptoms of the disorder or the predisposition toward the disorder. In an embodiment the treatment alleviates or relieves fibrosis. In an embodiment the treatment prevents at least one symptom of the disorder or to delays onset of at least one symptom of the disorder. The affect is beyond what is seen in the absence of treatment.

As used herein, an amount of a compound effective to treat a disorder, or a "therapeutically effective amount" refers to an amount of the compound which is effective, upon single or multiple dose administration to a subject, in treating a cell, to achieve treatment.

As used herein, an amount of a compound effective to prevent a disorder, or "a prophylactically effective amount" of the compound refers to an amount effective, upon single-or multiple-dose administration to the subject, in preventing or delaying the occurrence of the onset or recurrence of a disorder or a symptom of the disorder.

As used herein, the term "subject" is intended to include human and non-human animals. Exemplary human subjects include a human patient having a disorder, e.g., a disorder described herein or a normal subject. The term "non-human animals" of the invention includes all vertebrates, e.g., non-mammals (such as chickens, amphibians, reptiles) and mammals, such as non-human primates, domesticated and/or agriculturally useful animals, e.g., sheep, dog, cat, cow, pig, etc. In an embodiment the animal is other than a rodent, e.g., a rat or mouse, or a non-human primate.

### E. TITRATION OF A PATIENT:

Treatment of subjects can be optimized by titrating the subject, for example, such that treatment can be initiated with sub-optimal or no-effect doses of each compound and increased to determine the optimal dose of (6aR,10aR)-4-(1,1-dimethylheptyl)-Δ8-tetrahydrocannabinol-9-carboxylic acid or other (3R,4R)-Δ8-tetrahydrocannabinol-11-oic acid for the treatment and/or prevention of fibrotic diseases in the subject..

Treating a subject with (6aR,10aR)-4-(1,1-dimethylheptyl)-Δ8-tetrahydrocannabinol-9-carboxylic acid or other (3R,4R)-Δ8-tetrahydrocannabinol-11-oic acid can cause side effects such as dizziness, dry mouth, disorientation, euphoria, headache, nausea, pallor, somnolence, and vomiting.

The side effects can be modulated to some extent by starting at a low dose and slowly titrating the dose upward, e.g., during the course of treatment, for example over the course of weeks, months or years.

In some embodiments, a patient is titrated to minimize the adverse events and achieve a therapeutic level of the appropriate dosage form of (6aR,10aR)-4-(1,1-dimethylheptyl)-Δ8-tetrahydro-cannabinol-9-carboxylic acid or other (3R,4R)-Δ8-tetrahydrocannabinol-11-oic acid.

### F. KITS

A dosage form described herein may be provided in a kit. The kit includes (a) a compound used in a method described herein, and, optionally (b) informational material. The informational material can be descriptive, instructional, marketing or other material that relates to the methods described herein and/or the use of the dosage form for the methods described herein.

The informational material of the kits is not limited in its form. In one embodiment, the informational material can include information about production of the compound, molecular weight of the compound, concentration, date of expiration, batch or production site information, and so forth. In one embodiment, the informational material relates to methods for administering the compound.

In one embodiment, the informational material can include instructions to use a compound described herein in a suitable manner to perform the methods described herein, e.g., carry out a reaction to produce a compound described herein.

The informational material of the kits is not limited in its form. In many cases, the informational material, e.g., instructions, is provided in printed matter, e.g., a printed text, drawing, and/or photograph, e.g., a label or printed sheet. However, the informational material can also be provided in other formats, such as Braille, computer readable material, video recording, or audio recording. In another embodiment, the informational material of the kit is contact information, e.g., a physical address, email address, website, or telephone number, where a user of the kit can obtain substantive information about a compound described herein and/or its use in the methods described herein. Of course, the informational material can also be provided in any combination of formats.

In addition to a dosage form described herein, the composition of the kit can include other ingredients, such as a solvent or buffer, a stabilizer, a preservative, a flavoring agent (e.g.,a bitter antagonist or a sweetener), a fragrance, a dye or coloring agent, for example, to tint or color one or more components in the kit, or other cosmetic ingredient, and/or a second agent for treating a condition or disorder described herein. Alternatively, the other ingredients can be included in the kit, but in different compositions or containers than a compound described herein. In such embodiments, the kit can include instructions for admixing a compound described herein and the other ingredients, or for using a compound described herein together with the other ingredients.

In some embodiments, the components of the kit are stored under inert conditions (e.g.,under Nitrogen or another inert gas such as Argon). In some embodiments, the components of the kit are stored under anhydrous conditions (e.g., with a desiccant). In some embodiments, the components are stored in a light blocking container such as an amber vial.

A dosage form described herein can be provided in any form, e.g., liquid, dried or lyophilized form. It is preferred that a compound described herein be substantially pure and/or sterile. When a compound described herein is provided in a liquid solution, the liquid solution preferably is an aqueous solution, with a sterile aqueous solution being preferred. When a compound described herein is provided as a dried form, reconstitution generally is by the addition of a suitable solvent. The solvent, e.g., sterile water or buffer, can optionally be provided in the kit.

The kit can include one or more containers for the composition containing a dosage form described herein. In some embodiments, the kit contains separate containers, dividers or compartments for the composition and informational material. For example, the composition can be contained in a bottle, vial, or syringe, and the informational material can be contained in a plastic sleeve or packet. In other embodiments, the separate elements of the kit are contained within a single, undivided container. For example, the dosage form is contained in a bottle, vial or syringe that has attached thereto the informational material in the form of a label. In some embodiments, the kit includes a plurality (e.g., a pack) of individual containers, each containing one or more unit dosage forms (e.g., a dosage form described herein) of a compound described herein. For example, the kit includes a plurality of syringes, ampules, foil packets, or blister packs, each containing a single unit dose of a dosage form described herein.

The containers of the kits can be air tight, waterproof (e.g., impermeable to changes in moisture or evaporation), and/or light-tight.

The kit optionally includes a device suitable for use of the dosage form, e.g., a syringe, pipette, forceps, measured spoon, swab (e.g., a cotton swab or wooden swab), or any such device.

Thus, specific compositions and methods of treating fibrotic diseases using tetrahydrocannabinol-11-oic acids have been disclosed. A number of embodiments of the invention have been described. Nevertheless, it will be understood that various modifications may be made without departing from the spirit and scope of the invention. Accordingly, other embodiments are within the scope of the following claim. It should be apparent, however, to those skilled in the art that many more modifications besides those already described are possible without departing from the inventive concepts herein. The inventive subject matter, therefore, is not to be restricted except in the spirit of the disclosure. Moreover, in interpreting the disclosure, all terms should be interpreted in the broadest possible manner consistent with the context. In particular, the terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

All publications mentioned herein are incorporated herein by reference to disclose and describe the methods and/or materials in connection with which the publications are cited. The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates, which may need to be independently confirmed.

### EXPERIMENTAL

The following examples are provided in order to demonstrate and further illustrate certain preferred embodiments and aspects of the present invention and are not to be construed as limiting the scope thereof.

### EXAMPLE 1

### THE SYNTHETIC CANNABINOID AJULEMIC ACID EXERTS POTENT ANTI-FIBROTIC EFFECTS IN EXPERIMENTAL MODELS OF SYSTEMIC SCLEROSIS

### INTRODUCTION

Cannabinoids play key-roles in several - biological processes including inflammation, immunomodulation, and vasomotor response.[3, 4] Moreover, the cannabinoid system might also be implicated in the pathogenesis of fibrosis [5, 6]. The endocannabinoid system comprises the two specific cannabinoid receptors, CB1 and CB2, their endogenous ligands, and the machinery dedicated to endocannabinoid synthesis and degradation [7]. In experimental models of dermal fibrosis, the CB1 and CB2 cannabinoid receptors modulate fibrogenesis by abrogating the underlying inflammation [8, 9]. In addition, cannabinoid agonists are able to limit extracellular matrix (ECM) production by disrupting the TGF-beta cascade, and downregulating proliferation and activation of dermal fibroblasts [10, 11]. These data argue for a direct role of cannabinoids in limiting fibrosis, independently from their anti-inflammatory and immunomodulatory effects. However, the precise molecular mechanisms remain to be elucidated.

Currently, cannabinoids are not accepted as therapeutic agents due to their psychoactive effects. There is growing interest in the development of synthetic compounds without cannabimimetic activity on the central nervous system. Ajulemic acid (1,1'- dimethylheptyl-THC-11-oic acid) is a synthetic analog of tetrahydrocannabinol, devoid of relevant psychotropic effects [12]. It is a potent anti-inflammatory and analgesic agent in vivo [12]. First data in humans indicate that AjA is well tolerated and can reduce chronic neuropathic pain without significant psychotropic effects in doses up to 80 mg/day [13]. Although, AJA is able to activate CB1-mediated central effects by crossing the blood-brain barrier, the amount crossed seems not to be sufficient to trigger psychoactivity in humans [14, 15]. In addition to its affinity for CB1 and CB2 receptors, AjA also binds to and activates the peroxisome proliferated-activated receptor-y (PPAR-γ) [16]. PPAR-γ can be activated by some endocannabinoids and, in turn, PPAR-γ activation can modulate the endocannabinoid system, suggesting a reciprocal relationship [17]. Indeed, the agonist effects of endocannabinoids on PPAR-γ contribute to the regulatory effects of endocannabinoids on inflammation and vasoactivity [12].

Moreover, recent evidence suggests a key role for PPAR-γ in fi brogenesis. Suppression of PPAR-γ may contribute to the uncontrolled activation of fibroblasts [18]. In fact, PPAR-γ and its natural occurring ligand 15-deoxy-Δ12,14-prostaglandin J2 (15d-PGJ2), exert antifibrotic effects by suppressing collagen production and fibroblast activation in bleomycin-induced fibrosis [19, 20].

Systemic sclerosis (SSc) is an autoimmune disease in which vascular injury and inflammation lead to a progressive fibrosis of tissues [21]. Indeed, suppression of PPAR-γ may contribute to the uncontrolled activation of fibroblasts in SSc [18]. In fact, PPAR-γ and its natural occurring ligand, 15-deoxy-Δ12,14-prostaglandin J2 (15d-PGJ2), exert anti-fibrotic effects by suppressing collagen production and fibroblast activation in bleomycin induced models of fibrosis in mice [19, 20]. Therefore, cannabinoid receptors and PPAR-γ have been suggested as potential therapeutic targets in SSc. Considering that therapeutic doses of AjA simultaneously activate PPAR-γ and cannabinoid receptors without affecting the CNS, it could be hypothesized that AjA might also be effective in preventing fibrosis in experimental models of SSc.

### MATERIALS AND METHODS

### BLEOMYCIN-INDUCED DERMAL FIBROSIS

Skin fibrosis was induced in two groups of 6-week old DBA/2J mice by local injection of bleomycin. Briefly, 100 microliter of BLM dissolved in 0.9% NaCI at a concentration of 0.5 mg/ml were administered every other day, in well defined areas (1cm²) of the upper back. One group of BLM-challenged mice received orally AjA 1mg/kg/day, dissolved in seed oil. Subcutaneous injections of 100 ul 0.9% NaCI were used as controls. After 21 days, animals were killed by cervical dislocation. The injected skin was removed and processed for analysis. Each treatment group consisted of eight mice. The local ethical committee approved all animal experiments [22].

To test AjA on established fibrosisless dependent on inflamatory mechanisms, a modifi ed model of bleomycin-induced dermal fibrosis was assessed,₂₄ in which mice were challenged with bleomycin for 6 weeks and AjA treatment started during the last 3 weeks of bleomycin injections. Briefly, one group of mice was killed after receiving subcutaneous bleomycin injections for 6 weeks. The second group received bleomycin injections for 3 weeks followed by 0.9% NaCl injections for the next 3 weeks, to control for spontaneous regression of fi brosis. The third group was challenged with bleomycin for 6 weeks and was treated in parallel with AjA (1 mg/kg/day; oral administration) during the fi nal 3 weeks of bleomycin injections. Two groups of mice, receiving subcutaneous injections of 100 µl 0.9% NaCl for 3 weeks and 6 weeks, respectively, were used as controls. The injected skin was removed and processed for analysis.

To assess the effect of AjA in a different model of scleroderma fibrosis, a TGFβ-dependent model of dermal fibrosiswas studied. Dermal fibrosiswas induced in pathogen-free male C57BL/6 mice by intracutaneous injections of attenuated type V adenoviruses overexpressing a constitutively active TGFβ receptor I (AdTGFbRI) as described [23]. Three groups of mice were analysed. One group was treated in parallel orally with AjA (1 mg/kg/day) for the last 4 weeks. Mice infected with attenuated type V adenoviruses encoding only for LacZ and sham treated with the solvent of AjA served as controls. Animals were killed after 8 weeks by cervical dislocation and back skin was removed for analysis. Each treatment group consisted of eight mice.

### HISTOLOGICAL ANALYSIS

Three lesional skin samples were taken from every animal of each group and 5 µm skin sections (three for each skin sample) were stained with haematoxylin and eosin. Dermal thickness was calculated at 10x microscopic magnification by measuring the distance between the dermal-epidermal junction and the dermal-subcutaneous fat junction (micrometer) in five randomly selected fields for each skin section. Results were expressed as mean±SD. Two different examiners performed the evaluation blindly [23].

### DETERMINATION OF THE HYDROXYPROLINE CONTENT

The collagen content of lesional skin was evaluated by colourimetric quantification of hydroxyproline on three different skin biopsies (3 mm punch) taken from every animal in each group. Absorbencies were measured at 560 nm in triplicate. The results were expressed as µg of hydroxyproline per biopsy (mean±SD) [24].

### DETECTION OF MYOFIBROBLASTS

Skin sections (5 µm) were processed in order to evaluate expression of a smooth muscle actin (a-SMA) (mouse monoclonal antibody-Santa Cruz Biotechnology, Santa Cruz, California, USA) After deparaffinization, skin sections were incubated with 3% bovine serum albumin for 30 minutes to block nonspecific binding, followed by incubation with 3% H202 for 5 minutes to block endogenous peroxidase activity. α-SMA staining was performed according to the protocol of manufacturer using M.O.M kit (Vector, UK). Staining was visualized with diaminobenzidine, using a peroxidase substrate kit (Dako, Glostrup Denmark). Cell counts were performed blindly by two different operators, at 40x magnification, in five non-contiguous microscope fields of three sections from each lesional skin sample. Results are expressed as mean±SD of positive spindle-shaped fibroblastic cells per field [25].

### PATIENTS AND FIBROBLAST CULTURES

Fibroblast cultures were obtained from biopsy specimens of affected skin from 5 patients with SSc. Dermal fibroblasts were obtained by a 5 mm punch biopsy from the leading edge of the affected skin on the forearm of five patients affected by diffuse cutaneous SSc (dcSSc) in the fibrotic phase. All patients fulfilled the criteria for SSc classification proposed by LeRoy et al. [26] No patient was treated with animmunosuppressive agent or corticosteroids, nor taking cannabinoids for either recreational or therapeutic use at the time of biopsy (Table 1). Control fibroblasts were obtained from skin biopsy specimens from 5 healthy age- and sex-matched volunteers. Skin fibroblasts were expanded by outgrowth culture in Dulbecco's modified Eagle's medium (DMEM; Gibco Invitrogen) as described previously [27]. Fibroblasts from passages 3-6 were used for the experiments. All patients and healthy volunteers provided written informed consent using forms approved by the local institutional review boards.

**Table 1 Clinical and demographic characteristics of the patients with diffuse cutaneous systemic sclerosis (dcSSc)***

| **Characteristics** | **Value** |
|---|---|
| Sex (M/F), n | 1/4 |
| Age, median (range), years | 52 (35-72) |
| Disease duration, median (range), years† | 6 (3-12) |
| Diffuse cutaneous disease | 5 |
| Number of Scl-70 positive | 5 |
| Potential disease-modifying agents | 0 |
| Prostacyclin analogues | 5 |
| Calcium channel blockers | 4 |
| Prokinetics | 4 |

| | |
|---|---|
| *dcSSc was determined according to LeRoy et al criteria [28]. † From the first non-Raynaud's manifestation. | |

### STIMULATION EXPERIMENTS

SSc and healthy dermal fibroblasts were cultured in DMEM containing 2.5% of fetal calf serum (FCS) for 24 hours before the experiments. Dermal fibroblasts were incubated with AjA (JB Therapeutics, Newton, MA, USA), dissolved in dimethyl sulfoxide at 0.1, 1, 5 and 10microM concentrations for 24 hours. Experiments with the PPAR-γ irreversible antagonist GW9662 (Tocris Bioscience, Bristol, UK), 1 and 10 microM, were performed by incubating dermal fibroblasts for 15 minutes before AjA treatment.

### CELL VIABILITY

SSc and healthy fibroblasts were plated at a density of 100,000 cells/well in 24 well plate and treated with AjA at concentrations of 0.1, 1, 5 and 10 µM for 24 hours in the presence or absence of GW9662 at concentrations of 1 and 10 µM. Cell metabolic activity was measured by 3-(4,5-dimethylthiazol-2-yl)-2,5-diphenyltetrazolium bromide (MTT) assay. After removal of 100 µl of the growth medium, MTT was added at a final concentration of 3 mg/ml, and the cells were incubated at 37°C for an additional 4 hours. Formazan crystals were dissolved in DMSO and optical density was measured at 570 nm using a spectrophotometer [29].

### DETERMINATION OF THE COLLAGEN CONCENTRATION IN THE SUPERNATANT

The supernatant was collected and stored at -20°C prior to and after AjA ± GW9662 treatment. An EIA kit (Takara Bio, Otsuka, Japan) and ELISA kit (Euroclone, Lugano, Switzerland) were used to assess the procollagen type I carboxy-terminal peptide (PIP) and TGF-β and PGJ2 supernatant levels, respectively. Results are mean±SD of five separate experiments.

### WESTERN BLOT ANALYSIS

After rinsing twice with PBS, cells were lysed with Ripa buffer (Santa Cruz Biotechnology, Santa Cruz, California, USA). Subsequently, cell lysates were incubated on ice for 30 minutes and centrifuged at 14,000g for 20 minutes. Protein concentrations were measured using the Bradford assay (Bio-Rad, Reinach, Switzerland). Fifteen micrograms of protein from each sample was separated by SDS-10% polyacrylamide gel electrophoresis and electrotransferred onto nitrocellulose membranes according to standard protocols [30]. After blocking with 1% nonfat milk powder and 1% BSA for 1 hour, immunoblots were incubated with monoclonal antibodies against PPAR-γ (Cell Signalling, Danvers, USA) at a dilution of 1:200 overnight at 4°C. After incubation with horseradish peroxidase (HRP)-conjugated donkey anti-goat antibodies Santa Cruz Biotechnology) at a dilution of 1:10000 for 45 minutes, signals were detected with ECL Western Blotting Detection Reagents (Amersham Bioscience, Freiburg, Germany) and exposure to x-ray film (SuperRX; Fuji, Dusseldorf, Germany). For confirmation of equal loading of proteins, the amount of alpha-tubulin was determined using mouse anti-human a-tubulin antibodies (dilution 1:1,000; Sigma) and HRP-conjugated rabbit anti-mouse antibodies (dilution 1:5000; Santa Cruz). Results are mean±SD of three separate experiments.

### STATISTICAL ANALYSIS

Data are expressed as the mean±SD. Analysis of variance (ANOVA) was used to compare multiple means, followed by the Student-Newman Keuls post-hoc test (Sigma Stat V.3.5; Sigma Stat, Ashburn, Virginia, USA). P values less than 0.05 were considered significant.

### RESULTS

### AJULEMIC ACID PREVENTED BLEOMYCIN-INDUCED DERMAL FIBROSIS IN VIVO

To evaluate the potential of AjA as an anti-fibrotic agent in vivo, its efficacy in bleomycin-induced dermal fibrosis was tested. Animals were treated with bleomycin alone (BLM group; n=8), bleomycin plus AjA 1 mg/kg/day (BLM/AjA group; n=8) and 0.9% NaCI saline solution (control group; n=8). After treatment, the BLM group and BLM/AjA group did not differ in terms of body weight, and no other signs of toxicity of AjA such as ruffled fur or reduced activity were recorded.

Skin fibrosis was quantified at day 21 by determining dermal thickness and hydroxyproline content of lesional skin. Skin from BLM group showed dense accumulation of ECM in the dermis and accumulation of inflammatory cells in the deeper dermal layers and perivascular spaces. In addition, the subcutaneous fat tissue was largely replaced by connective tissue Figure 4A.

Two fold increase in dermal thickness was observed in mice injected with BLM compared with controls (p<0.001). Oral administration of AjA (lmg/kg/day) prevented development of skin fibrosis, and reduced skin thickness nearly to control levels (p<0.001 compared to mock treated mice) Figure 4B. Furthermore, in the BLM/AjA group the subcutaneous leukocyte infiltration, the accumulation of ECM and the fat layer replacement were reduced Figure 4A.

Consistent with the histology, production of collagen, determined by quantification of dermal hydroxyproline content, was reduced substantially in mice treated with AjA Figure 4C. In the BLM group, levels of hydroxyproline were more than two-fold greater than controls (p<0.001). AjA treatment (1 mg/kg/day) completely abrogated the collagen deposition induced by BLM (p<0.001).

Myofibroblasts, characterized by the cytoskeletal protein α-smooth muscle actin (a-sma), are considered one of the major cellular mediators of fibrosis in SSc. In BLM-challenged mice the number of α-SMA positive spindle-shaped fibroblastic cells per high-power field was increased two-fold compared with controls (p<0.001). AjA treatment significantly reduced the number of myofibroblasts in lesional skin up to 26±5% (mean ± SD) (p<0.05) Figure 4D.

### AJULEMIC ACID INHIBITED THE NEOSYNTHESIS OF COLLAGEN IN SSC FIBROBLASTS BY ACTIVATING PPAR-γ

To assess whether AjA is effective in an in vivo model of dermal fibrosis that is largely independent of autoimmune and infl ammatory phenomena, its efficacy in the constitutively active TGFβ receptor type I mouse model (AdTGFbRI) was tested. AdTGFbRI mice are characterised by generalized dermal fibrosis and fibroblast-specific activation of TGFβ signaling [22]. After 8 weeks AdTGFbRI mice showed twofold increase in dermal thickness compared with AdLacZ control mice (p<0.05). Oral administration of AjA (1 mg/kg/day) for the last 4 weeks reduced development of skin thickness up to 30±6% (mean±SD) compared with AdTGFbRI mice (p<0.05) (Figure 5A,B). AjA treatment (1 mg/kg/day) induced a mean reduction of 30±13% (mean±SD) in hydroxyproline content (p<0.05) (Figure 5C). AjA treatment also signifi cantly reduced the number of myofibroblasts in lesional skin up to 26±3% (p<0.001) (Figure 5D).

To further evaluate the efficacy of AjA for the treatment of established fibrosis, a 6- week model of bleomycininduced dermal fibrosis was used. Consistent with previous studies, dermal thickness increased by 76±4% (mean±SD) (p<0.05 vs NaCl controls) after 6 weeks of treatment with bleomycin. To assess the effects of AjA, mice challenged with bleomycin for 6 weeks were treated in parallel with AjA (1 mg/kg/day) during the final 3 weeks of bleomycin injections. Dermal thickness in mice treated with AjA for the last 3 weeks was reduced by 11±17% (mean±SD) compared with 6-week bleomycin-challenged mice (p<0.05). Furthermore, dermal thickness in mice treated with AjA did not differ signifi cantly from mice challenged with bleomycin for 3 weeks (Figure 6A, B). Consistently, a twofold increase of levels of hydroxyproline was recorded in the 6-week bleomycin-challenged group compared with controls (p<0.001). AjA treatment for the last 3 weeks reduced collagen deposition by 10±8% (mean±SD) (p<0.05). Thus, AjA administration for the last 3 weeks of bleomycin challenge stopped further progression of fi brosis, but did not alter pre-existing ECM accumulation (Figure 6C). The number of myofibroblasts after AjA treatment was signifi cantly reduced in lesional skin up to 25±16% (mean±SD) compared with bleomycin-challenged mice (p<0.05) (Figure 6D).

### AJULEMIC ACID INHIBITED COLLAGEN NEOSYNTHESIS IN DCSSC FIBROBLASTS THROUGH PPAR-γ

To investigate whether AjA directly inhibits collagen synthesis in cultured fibroblasts, the levels of procollagen type I propeptide (PIP) was quantified in supernatant from dcSSc and healthy cultured fibroblasts treated with AjA. AjA (0.1 to 10 µM) reduced the levels of PIP released from dcSSc fibroblasts in a dose-dependent manner. Maximal inhibition was observed at 10 µM concentration with a mean PIP reduction up to 60±7% (p<0.001)

(Figure 7). Similar results were seen in healthy dermal fibroblasts (data not shown).

AjA did not exert toxic effects on fibroblasts at the concentrations used. The metabolic activity measured by the MTT assay was not affected by treatment with AjA in concentrations up to 10 µM (data not shown).

To evaluate a PPAR-γ pathway dependency, dcSSc and healthy fibroblasts treated with AjA were preincubated with the highly selective PPAR-γ antagonist GW9662 (1-10 µM), The inhibitory effect of AjA on collagen production was completely prevented by GW9662 at 10 µM concentration (p<0.05) (Figure **8**A).

In addition, the effect of AjA on expression of the endogenous PPAR-γ ligand 15d-PGJ2 was analysed. The levels of 15d-PGJ2 were reduced in supernatants of dcSSc fibroblasts compared with controls (p<0.001). However, incubation with AjA strongly increased the levels of 15d-PGJ2 in a dose-dependent manner (p<0.001) (Figure **8**B).

Further, PPAR-γ expression in dcSSc and healthy fibroblasts was assessed. The protein levels of PPAR-γ were reduced in dcSSc fibroblasts compared with healthy controls. However, treatment with AjA (5 µM and 10 µM) significantly increased the expression of PPAR-γ in dcSSc fibroblasts (p<0.001) (Figure **8**C).

(TGF) is considered a major profi brotic cytokine in orchestrating the uncontrolled activation of SSc fibroblasts with consequent overproduction of ECM. Moreover, in fibroblasts, TGFβ signalling is inversely related to PPAR-γ physiology function [31]. Since AjA suppressed collagen production through PPAR-γ activation, and in vivo reduced dermal fibrosisin the constitutively active TGFβ receptor type I mouse model (AdTGFbRI), its ability to downregulate TGFβ in vitro was evaluated. TGFβ concentrations in supernatants from dcSSc fibroblasts were signifi cantly higher than TGFβ concentrations in cultures from healthy controls (p<0.001). Upon incubation with AjA, a significant dose-dependent reduction in TGFβ concentrations (up to 50%) was seen in supernatants from dcSSc fibroblasts (p<0.001), with maximum suppression seen at 2 h (Figure 12).

To investigate, whether AjA directly inhibits the collagen synthesis in cultured fibroblasts, the levels of procollagen type I propeptide (PIP) in supernatant from SSc was quantified and healthy cultured fibroblasts treated with AjA. AjA dose-dependently decreased the levels of PIP released from fibroblasts in concentrations from 0.1 to 10 µM. Maximal inhibition was observed at 10 µM concentration with a mean PIP reduction up to 60% (p<0.001) Figure 12A Similar results were observed in healthy dermal fibroblasts (data not shown).

AjA did not exert toxic effects on fibroblasts in the concentrations used herein. The metabolic activity measured by the MTT assay was not affected by treatment with AjA in concentrations up to 10 microM (data not shown).

To evaluate a PPAR-γ pathway dependency, SSc and healthy fibroblasts treated with AjA were pre-incubated with the highly selective PPAR-γ antagonist GW9662 (1-10 µM). Interestingly, the inhibitory effect of AjA on collagen production was prevented by GW9662 in a dose-dependent manner. In concentrations of 10 microM, GW9662 completely prevented the anti-fibrotic effects of AjA (p<0.05) Figure 11. In addition, PPAR-γ expression in SSc and healthy fibroblasts were assessed. The protein levels of PPAR-γ were reduced in SSc fibroblasts compared to healthy controls.

However, treatment with AjA (5microM and 10microM) completely restored the expression of PPAR-γ and increased the levels of PPAR-γ in SSc fibroblasts to the levels in fibroblasts from healthy volunteers (p<0.001).(Figure 12A-B) Further, the effect of AjA on the expression of the endogenous PPAR-γ ligand 15d-PGJ2 production were analyzed. The levels of 15d-PGJ2 were reduced in the supernatants of SSc fibroblasts compared to controls (p<0.001). However, incubation with AjA strongly increased the levels of 15d-PGJ2 in dose-dependent manner (p<0.001). (Figure 11)

### AJULEMIC ACID INHIBITED TGF-β PRODUCTION

Transforming growth factor-beta (TGF-beta) is considered a major profibrotic cytokine in orchestrating the uncontrolled activation of SSc fibroblasts with consequent overproduction of ECM. Since AjA suppresses collagen production, its capability to downregulate TGF-beta was evaluated. TGF-beta concentrations in supernatants from SSc fibroblasts were significantly higher than TGF-beta concentrations in cultures from healthy controls (p<0.001). Upon incubation with AjA, a significant, dose-dependent reduction in TGF-beta concentrations (up to 50%) was observed in supernatants from SSc fibroblasts (p<0.001) with maximum suppression observed at 2 hr Figure 12B.

### DISCUSSION

Results of experiments described here show that AjA efficiently prevents bleomycin-induced dermal fibrosis in mice. In SSc fibroblasts, AjA inhibits collagen synthesis through PPAR-γ agonism.

Modulation of the CB1 and CB2 receptors limit skin fibrosis by reducing the upstream inflammation [8, 9]. Although the synthetic THC analog AjA, is a ligand of the classical cannabinoids receptors CB1 and CB2 [12], the data be suggest that AjA may also act to reduce fibrosis directly through a PPAR-γ mediated mechanism. Consistent with this notion are, data which indicate that the high affinity cannabinoid receptor agonist WIN55,212-2 exerts anti-fibrotic effects that are not mediated by the classic cannabinoid receptors [10, 11]. A close relationship between the endocannabinoid system and PPAR-γ signaling has been postulated [17]. Accumulating evidence suggests that impaired expression and function of PPAR-γ is important to the pathogenesis of fibrosis in SSc [31]. Such diminished activity of PPAR signaling is counterbalanced by an increase of the SMAD-mediated and SMADindependent TGF beta cascade leading to fibrosis [30]. It has been suggested that cross-talk between these two mediators in physiologic conditions orchestrate the mechanisms taking place along with the healing processes [18].

Consistent with these findings, it was observed that the increased release of TGF-beta is mirrored by a concomitant decrease of PPAR-γ. The potent stimulatory effects of AjA on PPAR-γ might be mediated by direct and also by indirect effects. It was demonstrated that AjA reduces the release of TGF-beta from SSc fibroblasts. Moreover, AjA also stimulates the expression of the endogenous PPAR-γ ligand PGJ2. By inhibiting TGF-beta and stimulating the release of PGJ2, AjA completely restores PPAR-γ signaling in SSc fibroblasts and increases PPAR-γ to levels observed in fibroblasts from healthy volunteers.

It was also shown that oral administration of AjA attenuates markedly the dermal fibrosis induced in DBA/2J mice by bleomycin. AjA reduces fibroblast activation, ECM deposition, and subsequent dermal thickening. Bleomycin-induced dermal fibrosis is considered a reliable experimental model for SSc that reflects the early stages of the disease, with increased deposition of collagen and other ECM components, migration of inflammatory cells into the skin, and atrophy of the adipose tissue [32]. It is still unclear why in SSc atrophy of the fat tissue occurs, in favour of connective tissue replacement. However, recent data suggest that PPAR-γ might play a central role in regulating the balance between adipogenesis and fibrogenesis [18]. Accumulating evidence suggests that increased activation of PPAR-γ favours adipogenesis, whereas decreased PPAR-γ favors tissue fibrosis. According to this model, the downregulation of PPAR-γ observed in SSc would enhance the recruitment of mesenchymal precursors, stimulate the release of collagen from resident fibroblasts, and inhibit adipogenic differentiation [18]. Consistent with this hypothesis, AjA stimulates the differentiation of embryogenic fibroblastic 3T3-L1 cells into adipocytes, a process that is known to be mediated by PPAR-γ [12]. Based on these results, it could be suggested that AjA might exert its anti-fibrotic effects by restoring the defective PPAR-γ activation in SSc fibroblasts. Consistent with this hypothesis, it was observed that AjA stimulates the expression of PPAR-γ in SSc fibroblasts by upregulating the expression of its endogenous ligand 15dPGJ2. Moreover, it is shown that the activation of PPAR-γ is essential for the anti-fibrotic effects of AjA and that inhibition of PPAR-γ completely abrogates the inhibitory effects of AjA on collagen synthesis. Moreover, treatment of bleomycin challenged mice with AjA not only prevents fibrosis, but also the characteristic atrophy of the subcutis.

Results of the studies presented in this paper show that AjA reduces infiltration of the dermis by inflammatory cells. The possibility cannot be excluded that the anti-inflammatory effects of AjA might also contribute to its anti-fibrotic activity in vivo, in particular because the mouse model of bleomycin induced fibrosis is responsive to anti-inflammatory drugs [22]. However, it was also observed potent direct anti-fibrotic effects of pharmacologically relevant concentrations of AjA on cultured fibroblasts in the absence of inflammatory cells. Further in vivo studies with less inflammation dependent models of SSc will help to dissect further the relative contributions of direct anti-fibrotic effects on fibroblasts and indirect anti-fibrotic effects mediated via inhibition of inflammatory cells.

In conclusion, it was demonstrated that AjA exerts potent anti-fibrotic effects in vitro and in vivo by stimulating PPAR-γ signaling. Cannabinoids as well as PPAR-γ agonists might be ideal drugs targeting SSc, since they can modulate fibrosis, inflammation, and vasodilatation, all of which are dysregulated in SSc. These findings also might have direct translational implications because therapeutic doses of AjA are well tolerated in humans without unwanted effects on the central nervous system.

### REFERENCES:

1. Stahl, P. H. and Wermuth, C. G., (Eds.) (2002) Handbook of Pharmaceutical Salts: Properties Selection and Use, Verlag Helvetica Chimica Acta/Wiley-VCH, Zurich.
2. LeRoy, E. C. (1974) Increased Collagen Synthesis by Scleroderma Skin Fibroblasts in Vitro a Possible Defect in the Regulation or Activation of the Scleroderma Fibroblast, J. Clin. Invest. 54(4), 880-889.
3. Klein, T. W. (2005) Cannabinoid-based drugs as anti-inflammatory therapeutics, Nat. Rev. Immunol. 5(5), 400-411.
4. Pertwee, R. G. (2009) Emerging strategies for exploiting cannabinoid receptor agonists as medicines, Br. J. Pharmacol. 156(3), 397-411.
5. Teixeira-Clerc, F. et al. (2008) Le système endocannabinoïde, une nouvelle cible pour le traitement de la fibrose hépatique, Pathol. Biol. 56(1), 36-38.
6. Michalski, C. et al. (2008) Cannabinoids reduce markers of inflammation and fibrosis in pancreatic stellate cells, PLoS One 3(2), e1701.
7. Pertwee, R. G. (2005) Pharmacological actions of cannabinoids, Handb. Exp. Pharmacol. (168), 1-51.
8. Akhmetshina, A. et al. (2009) The cannabinoid receptor CB2 exerts antifibrotic effects in experimental dermal fibrosis, Arthritis. Rheum. 60(4), 1129-1136.
9. Marquart, S. et al. (2010) Inactivation of the cannabinoid receptor CB1 prevents leukocyte infiltration and experimental fibrosis, Arthritis. Rheum. 62(11), 3467-3476.
10. Servettaz, A. et al. (2010) Targeting the Cannabinoid Pathway Limits the Development of Fibrosis and Autoimmunity in a Mouse Model of Systemic Sclerosis, The American Journal of Pathology 177(1), 187-196.
11. Balistreri, E. et al. (2011) The cannabinoid WIN55, 212-2 abrogates dermal fibrosis in scleroderma bleomycin model, Ann. Rheum. Dis. 70(4), 695-699.
12. Burstein, S. (2005) Ajulemic acid (IP-751): Synthesis, proof of principle, toxicity studies, and clinical trials, AAPS J. 7(1), E143-E148.
13. Salim, K. et al. (2005) Pain measurements and side effect profile of the novel cannabinoid ajulemic acid, Neuropharmacology 48(8), 1164-1171.
14. Vann, R. E. et al. (2007) Cannabimimetic Properties of Ajulemic Acid, J. Pharmacol. Exp. Ther. 320(2), 678-686.
15. Karst, M. (2007) Comments on "Cannabimimetic Properties of Ajulemic Acid", J. Pharmacol. Exp. Ther. 322(1), 420-421.
16. Liu, J. et al. (2003) Activation and Binding of Peroxisome Proliferator-Activated Receptor γ by Synthetic Cannabinoid Ajulemic Acid, Mol. Pharmacol. 63(5), 983-992.
17. O'Sullivan, S. E. (2007) Cannabinoids go nuclear: evidence for activation of peroxisome proliferator-activated receptors, Br. J. Pharmacol. 152(5), 576-582.
18. Wei, J. et al. (2010) PPARγ downregulation by TGFβ in fibroblast and impaired expression and function in systemic sclerosis: a novel mechanism for progressive fibrogenesis, PLoS One 5(11), e13778.
19. Genovese, T. et al. (2005) Effect of rosiglitazone and 15-deoxy-Δ12,14-prostaglandin J2 on bleomycin-induced lung injury, Eur. Respir. J. 25(2), 225-234.
20. Kapoor, M. et al. (2009) Loss of peroxisome proliferator-activated receptor γ in mouse fibroblasts results in increased susceptibility to bleomycin-induced skin fibrosis, Arthritis. Rheum. 60(9), 2822-2829.
21. Varga, J. and Abraham, D. (2007) Systemic sclerosis: a prototypic multisystem fibrotic disorder, J. Clin. Invest. 117(3), 557-567.
22. Beyer, C. et al. (2010) Animal models of systemic sclerosis: Prospects and limitations, Arthritis. Rheum. 62(10), 2831-2844.
23. Avouac, J. et al. (2012) Inhibition of activator protein 1 signaling abrogates transforming growth factor β-mediated activation of fibroblasts and prevents experimental fibrosis, Arthritis. Rheum. 64(5), 1642-1652.
24. Reich, N. et al. (2010) The transcription factor Fra-2 regulates the production of extracellular matrix in systemic sclerosis, Arthritis. Rheum. 62(1), 280-290.
25. Akhmetshina, A. et al. (2009) Treatment with imatinib prevents fibrosis in different preclinical models of systemic sclerosis and induces regression of established fibrosis, Arthritis. Rheum. 60(1), 219-224.
26. Subcommittee for scleroderma criteria of the American Rheumatism Association Diagnostic and Therapeutic Criteria Committee. (1980) Preliminary criteria for the classification of systemic sclerosis (scleroderma), Arthritis. Rheum. 23(5), 581-590.
27. Garcia-Gonzalez, E. et al. (2009) Cannabinoids inhibit fibrogenesis in diffuse systemic sclerosis fibroblasts, Rheumatology 48(9), 1050-1056.
28. Gonzalez, E. G. et al. (2012) Synthetic cannabinoid ajulemic acid exerts potent antifibrotic effects in experimental models of systemic sclerosis, Ann. Rheum. Dis.(E-pub April 4, 2012).
29. Venalis, P. et al. (2009) Lack of inhibitory effects of the anti-fibrotic drug imatinib on endothelial cell functions in vitro and in vivo, J. Cell. Mol. Med. 13(10), 4185-4191.
30. Kulkarni, A. A. et al. (2011) PPAR-γ ligands repress TGFβ-induced myofibroblast differentiation by targeting the PI3K/Akt pathway: implications for therapy of fibrosis, PLoS One 6(1), e15909.
31. Wei, J. et al. (2011) Fibrosis in systemic sclerosis: Emerging concepts and implications for targeted therapy, Autoimmun. Rev. 10(5), 267-275.
32. Yamamoto, T. (2010) Animal model of systemic sclerosis, The Journal of Dermatology 37(1), 26-41.

### ITEMS:

1. A method, comprising:
   a) providing:
      i) a subject exhibiting at least one symptom of fibrotic disease;
      ii) a composition comprising ajulemic acid;
   b) administering said composition to said subject; and
   c) reducing said at least one symptom of fibrotic disease.
2. The method according to item 1, wherein said fibrotic disease is dermal fibrosis and said symptom is dermal thickening.
3. The method according to item 1, wherein said fibrotic disease is lung fibrosis and said symptom is leukocyte infiltration.
4. The method according to item 1, wherein said fibrotic disease is selected from the group consisting of scleroderma, systemic sclerosis, scleroderma-like disorders, sine scleroderma, liver cirrhosis, interstitial pulmonary fibrosis, Dupuytren's contracture, keloids, chronic kidney disease, chronic graft rejection, and other scarring/wound healing abnormalities, post operative adhesions, and reactive fibrosis.
5. The method according to item 1, wherein said composition is administered orally.
6. The method according to item 1, wherein said composition is administered intravenously.
7. The method according to item 1, wherein said composition is administered via an implant or patch.
8. The method according to item 7, wherein said implant or patch provides slow release of said composition.
9. The method according to item 1, wherein said composition is administered by inhalation.
10. The method according to item 1, wherein said composition is administered in a tablet.

## Claims

1. A pharmaceutical composition comprising ajulemic acid, or a pharmaceutically-acceptable salt thereof, for use in treating a fibrotic disease in a subject in need thereof, wherein the fibrotic disease is selected from keloids, chronic graft rejection, post-operative adhesions, and reactive fibrosis.

2. The pharmaceutical composition for the use of claim 1, wherein the fibrotic disease is keloids.

3. The pharmaceutical composition for the use of claim 1, wherein the fibrotic disease is chronic graft rejection.

4. The pharmaceutical composition for the use of claim 1, wherein the fibrotic disease is post-operative adhesions.

5. The pharmaceutical composition for the use of claim 1, wherein the fibrotic disease is reactive fibrosis.

6. The pharmaceutical composition for the use of any one of claims 1-5, wherein the pharmaceutical composition is formulated for oral administration.

7. The pharmaceutical composition for the use of claim 6, wherein the pharmaceutical composition is a tablet or a capsule.

8. The pharmaceutical composition for the use of claim 7, wherein the tablet comprises an enteric coating, comprises a semi-permeable coating, or is formulated for sustained release.

9. The pharmaceutical composition for the use of claim 7, wherein the capsule is formulated for osmotic release or is formulated for sustained release.

10. The pharmaceutical composition for the use of any one of claims 1-5, wherein the pharmaceutical composition is formulated for topical administration.

11. The pharmaceutical composition for the use of claim 10, wherein the pharmaceutical composition is a patch, gel, ointment, cream, or aerosol.

12. The pharmaceutical composition for the use of any one of claims 1-5, wherein the pharmaceutical composition is formulated for intravenous administration.

13. The pharmaceutical composition for the use of any one of claims 1-5, wherein the pharmaceutical composition is formulated to be administered by inhalation.

14. The pharmaceutical composition for the use of any one of claims 1-5, wherein the pharmaceutical composition comprises a unit dose of about 5 mg to about 120 mg ajulemic acid and is administered once daily.

15. The pharmaceutical composition for the use of any one of claims 1-5, wherein the pharmaceutical composition comprises a unit dose of about 2 mg to about 40 mg ajulemic acid and is administered up to three times daily.
